(19) Europäisches Patentamt / European Patent Office / Office européen des brevets

(11) EP 3 679 155 B1

(12) **EUROPEAN PATENT SPECIFICATION**

(45) Date of publication and mention of the grant of the patent:
**15.12.2021 Bulletin 2021/50**

(21) Application number: **18756472.9**

(22) Date of filing: **28.08.2018**

(51) Int Cl.:
***C12Q 1/6876*** *(2018.01)*

(86) International application number:
**PCT/EP2018/073150**

(87) International publication number:
**WO 2019/043015 (07.03.2019 Gazette 2019/10)**

(54) **METHOD TO CONFIRM VARIANTS IN NGS PANEL TESTING BY SNP GENOTYPING**

VERFAHREN ZUM BESTÄTIGEN DER VARIANTEN IN EINES NGS-PANEELTESTS DURCH SNP-GENOTYPISIERUNG

PROCÉDÉ POUR CONFIRMER DES VARIANTES DANS UN TEST DE PANNEAU NGS PAR GÉNOTYPAGE DE SNP

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.08.2017 EP 17306106**

(43) Date of publication of application:
**15.07.2020 Bulletin 2020/29**

(73) Proprietor: **Assistance Publique - Hôpitaux de Paris**
**75004 Paris (FR)**

(72) Inventors:
- **CAZENEUVE, Cécile**
  **69007 Lyon (FR)**
- **NOEL, Sandrine**
  **95500 Gonesse (FR)**

(74) Representative: **Regimbeau**
**20, rue de Chazelles**
**75847 Paris Cedex 17 (FR)**

(56) References cited:
**WO-A1-2014/082032 WO-A1-2016/022641**

- **GIBRAN HEMANI ET AL: "Detection and replication of epistasis influencing transcription in humans", NATURE, vol. 508, no. 7495, 26 February 2014 (2014-02-26), pages 249-253, XP055425456, ISSN: 0028-0836, DOI: 10.1038/nature13005**
- **MING LI ET AL: "P109 Validation of genotyping method for human leukocyte based on next generation sequencing technology", HUMAN IMMUNOLOGY, vol. 77, 26 September 2016 (2016-09-26), page 118, XP029710811, ISSN: 0198-8859, DOI: 10.1016/J.HUMIMM.2016.07.175**
- **JESSICA K. MILLER ET AL: "Use of Sequenom Sample ID Plus SNP Genotyping in Identification of FFPE Tumor Samples", PLOS ONE, vol. 9, no. 2, 13 February 2014 (2014-02-13), page e88163, XP055425460, DOI: 10.1371/journal.pone.0088163**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 679 155 B1

## Description

### TECHNICAL FIELD OF THE INVENTION

The present invention belongs to the field of methods to validate genotyping results obtained by Next-Generation Sequencing (NGS) for a series of patients, to detect sample mix-ups and prevent misdiagnosis. In particular, the present invention relates to a method to validate NGS genotyping results by genotyping Single Nucleotide Polymorphisms (SNPs) of a specific panel, adapted for allele-specific multiplex PCR, allowing accurate validation of NGS data by sample pairing. The present invention also relates to a kit comprising an optimized set of primers to detect this SNP panel and its use for validating NGS genotyping results.

### BACKGROUND ART

NGS refers to high throughput sequencing technologies in which clonally amplified DNA templates, or single DNA molecules, are sequenced in a massively parallel fashion in a flow cell. Sequencing is conducted in either a stepwise iterative process or in a continuous real-time manner. By virtue of the highly parallel process, each clonal template or single molecule is "individually" sequenced and can be counted among the total sequences generated. This has positioned NGS as the method of choice for largescale complex genetic analyses (Voelkerding et al., 2010).

However, NGS workflows are very complex and comprise multiple processing steps, such as library preparation, DNA sample quality control, amplification of sample library, sequencing and bioinformatics process. As a consequence of numerous liquid transfers, incubations, and purification steps as well as addition of index containing adapters - short single strand DNA sequences added at the end of the library fragments that allow identification of sample by sequencing —, sample mix-up are both possible and difficult to detect. However, in the framework of diagnosis of hereditary diseases, it is crucial to ascertain genotyping results for several reasons. First, genotyping results have consequences for genetic counseling and further molecular analyzes for the index case as well as for his family: the presence of mutation(s) in the index case must therefore be absolutely certain. Second, laboratory may identify genetic variation(s) of unknown significance at the time of the NGS analysis: this(ese) variant(s), which cannot be used for genetic counseling at this time, may, according to future published scientific data, be later interpreted as being polymorphism(s) or disease causing mutation(s). In this later case, this new interpretation has to be communicated to the index case, with the same consequences for genetic counseling and further analyzes than previously mentioned.

To validate NGS genotyping results and to identify possible sample mix-ups, various techniques are available, such as installation of barcoding for sample tracking (G. Matthijs et al., 2016) or Sanger sequencing, which is the most currently used method to confirm the mutations identified by NGS assay. However, this technic is very costly in terms of technician time and reagents, and is usually restricted to patients presenting with a disease causing mutation (not all patients in a series present with such mutation).

Panel of single nucleotide polymorphisms (SNPs) have been proposed to facilitate the validation of data provenance in whole-exome sequencing (WES) studies (Pengelly et al., 2013). These SNPs were preferentially selected in protein-coding regions of the genome, in particular in genes of clinical interest, which are targeted in WES studies. Therefore, the study of these SNPs can lead to the detection of unsolicited findings in the regions surrounding the SNPs, although they may nevertheless be suitable for use in an allele-specific multiplex PCR.

Hence, what is needed is a new reliable method for validating NGS genotyping results which is cost effective, easy to use and which reduces the risk of detection of unsolicited finding.

The applicant therefore found that validation of NGS genotyping results could be obtained by sample tracking consisting in the comparison of the genotype of a particular SNPs set obtained both by the NGS assay that provided the said NGS genotyping results and, independently, from the "primary" DNA samples by another method, hereinafter referred to as SNP profiling assay.

Hence, the present invention relates to a method to validate NGS genotyping results of a panel of genes tested in a series of at least 2 patients characterized in that said validation is provided by the SNP profiling assay. The NGS genotyping results, do not need to be confirmed by another technique if the results of the SNP profiling assay is strictly identical to the corresponding NGS genotyping results, and if there are not two patients from the series with identical SNP profiles. In contrast, when there are not two identical SNP profiles in the series of patients, results of SNP profiling assay not strictly identical to NGS genotyping results will reveal sample mix-up. In this case, further validation is necessary.

If two patients of the series have the same SNP profile, either they are really different people (NGS genotyping results showing many differences), or the same DNA sample has been mistakenly tested twice (identical NGS genotyping results for the two identifiers).

In the first case (identical SNP profiles but distinct NGS genotyping results), a sequencing assay (e.g. Sanger sequencing) would have to be subsequently performed to validate NGS genotyping results for both patients.

In the second case (same SNP profile and identical NGS genotyping results), biological samples from a unique patient have been mistakenly identified as originating from two different patients. Then, further validation is necessary: for instance, new biological samples for both patients need to be requested and tested (by any suitable method, in particular the same SNP profiling assay) to determine which one has been tested in NGS and SNP profiling assays.

## SUMMARY OF THE INVENTION

In the context of the present invention, the inventors surprisingly found that NGS genotyping results could be efficiently validated by sample tracking based on the comparison of a SNP profile, consisting in the genotype of a particular SNPs set, obtained both by the NGS assay that provided the said NGS genotyping results and, independently, from the "primary" DNA samples of tested patients, by SNP profiling assay. The SNPs are specifically selected according to the following features:

i. they are not located in a repeated sequence of the genome;
ii. they are biallelic;
iii. the 60 bases flanking sequences at either side of the SNP site has a GC content < 70% and an AT content <70%;
iv. they are not associated to a known pathology.

In a first aspect, the present invention thus relates to a method to validate NGS genotyping results of a panel of genes tested in series of at least 2 patients characterized in that said validation is provided by SNP profiling assay, said method comprising the steps of:

a) determining the genotype for a combination of at least 8 SNPs by an independent SNP profiling assay using the primary DNA samples used to obtain said NGS genotyping results, said NGS genotyping results including the genotype for said SNPs;
b) comparing the SNPs genotypes obtained by said SNP profiling assay and said NGS assay; and
c) validating or not NGS genotyping results based on said comparison, wherein:

1) If there are not two patients from the series with identical SNP profiles, and said SNPs genotypes obtained by said SNP profiling assay and said NGS assay are identical, then NGS genotyping results are validated; and
2) If two patients have identical SNP profiles but NGS genotyping results are distinct, a sequencing assay (e.g. Sanger sequencing) is further performed for these two patients, in order to validate their NGS genotyping results; and
3) In other cases, NGS genotyping results are not validated and further validation is necessary;

wherein said SNPs have the following features:

i. they are not located in a repeated sequence of the genome;
ii. they are biallelic;
iii. the 60 bases flanking sequences at either side of the SNP site has a GC content < 70% and an AT content <70%;
iv. they are not associated to a known pathology.

In a second aspect, a kit for detection of a combination of at least 8 SNPs according to the invention is provided, which comprises specific primers to detect said SNPs by allele-specific polymerase chain reaction (allele-specific PCR), and preferably further comprises PCR multiplex reagents, and/or NGS oligonucleotide probes or primers designed to capture or amplify sequences comprising said at least 8 SNPs.
In a third aspect, a method for detecting polymorphisms in the DNA of patients is provided, comprising performing, preferentially in parallel, the two following steps:

a) detecting polymorphisms by NGS assay, and
b) validating NGS genotyping results using the method according to the invention.

## DESCRIPTION OF THE FIGURES

**Figure 1:** Allele-specific PCR amplification using primers differentiating alleles 1 and 2 of each polymorphism by the size of the PCR products. A. PCR AS1: Hybridization of sense strand primer specific for allele 1 (Primer AS1) on allele 1 and allele 2 results in PCR product of n base pairs (bp) in size and no PCR product, respectively. PCR AS2: Hybridization of sense strand primer specific for allele 2 (Primer AS2) on allele 1 and 2 results in no PCR product and PCR product of n+3bp, respectively, the latter being generated in this embodiment by the addition of 3 bases to the 5'end of Primer AS2. B. Size of PCR products resulting from amplification using both Primers AS1 and AS2, according to the SNP genotype. +: presence; -: absence. Opposite strand primer is not represented.

**Figure 2:** Results (electrophoregrams) of SNP profiling assay for three patients. The genotype is determined for each SNP by the presence of: only one peak corresponding to allele 1 (genotype 1/1), or only one peak corresponding to allele 2 (genotype 2/2), or the presence of two peaks, corresponding to allele 1 and 2 (genotype 1/2).

## DETAILED DESCRIPTION OF THE INVENTION

In the context of the present invention, the inventors surprisingly found that NGS genotyping results could be efficiently validated by sample tracking based on the comparison of a SNP profile, consisting in the genotype of a particular SNPs set, obtained both by the NGS assay that provided the said NGS genotyping results and, independently, from the "primary" DNA samples of tested patients by SNP profiling assay. The SNPs are specifically selected according to the following features:

i. they are not located in a repeated sequence of the genome;
ii. they are biallelic;
iii. the 60 bases flanking sequences at either side of the SNP site has a GC content < 70% and an AT content <70%;
iv. they are not associated to a known pathology.

The present invention thus provides a method to validate NGS genotyping results of a panel of genes tested in series of at least 2 patients characterized in that said validation is provided by SNP profiling assay, said method comprising the steps of:

a) determining the genotype for a combination of at least 8 SNPs by an independent SNP profiling assay using the primary DNA samples used to obtain said NGS genotyping results, said NGS genotyping results including the genotype for said SNPs;
b) comparing the SNPs genotypes obtained by said SNP profiling assay and said NGS assay; and
c) validating or not NGS genotyping results based on said comparison, wherein:

   1) If there are not two patients from the series with identical SNP profiles, and said SNPs genotypes obtained by said SNP profiling assay and said NGS assay are identical, then NGS genotyping results are validated; and
   2) If two patients have identical SNP profiles but NGS genotyping results are distinct, a sequencing assay (e.g. Sanger sequencing) is further performed for these two patients, in order to validate their NGS genotyping results; and
   3) In other cases, NGS genotyping results are not validated and further validation is necessary;

wherein said SNPs have the following features:

i. they are not located in a repeated sequence of the genome;
ii. they are biallelic;
iii. the 60 bases flanking sequences at either side of the SNP site has a GC content < 70% and an AT content <70%;
iv. they are not associated to a known pathology.

The term "biological sample" refers to any sample that comprises nucleic acids, such as any tissue (biopsy for instance), or any type of cells (isolated or present in body fluid). Preferably, the biological sample is derived from a human or animal, preferably human. Preferably, the sample is selected from the group consisting of cells (healthy or not, e.g. tumor cells), tissue (e.g. organ tissue samples such as lung, kidney or liver) and body fluids (e.g. blood, blood products such as buffy coat, plasma and serum, urine, liquor, sputum, stool, CSF (cerebrospinal fluid) and sperm, epithelial swabs, biopsies, bone marrow samples). The term "biological sample" also includes processed samples such as preserved, fixed and/or stabilised samples. The term "biological sample" also includes artificial samples which comprise nucleic acids such as compositions comprising already purified nucleic acids.

By "primary DNA samples" it is meant DNA samples which are directly obtained from a biological sample of a patient, from which aliquots will be taken to perform, in parallel, the NGS assay and the SNP profiling assay. Preferentially, such primary DNA samples have not been amplified or diluted, but same limited transformation of the sample may have been performed (e.g. genomic DNA extraction or mRNA extraction followed by reverse transcription to obtain cDNA). The term "DNA" refers to genomic DNA or cDNA, preferentially genomic DNA (less transformation of biological sample and necessary if at least one SNP is not in coding regions).

*SNPs number and selection criteria*

As used herein, the term "single nucleotide polymorphism" or "SNP" refers to a polymorphic site occupied by a single nucleotide, which is the site of variation between allelic sequences. The site is usually preceded by and followed by highly conserved sequences of the allele (e.g., sequences that vary in less than 1/100 or 1/1000 members of the populations). A single nucleotide polymorphism usually arises due to substitution of one nucleotide for another at the polymorphic site. Single nucleotide polymorphisms can also arise from a deletion of a nucleotide or an insertion of a nucleotide relative to a reference allele. SNPs are common sequence variations in the human genome, and each individual has a unique combination of these nucleotide variations. "SNP profiling assay" means that for each primary DNA samples obtained from a patient, several SNPs are detected and combined to determine the combination, or profile, of these nucleotide variations. Thus, by "validate results of NGS" it is meant that SNP profiles obtained in the independent SNP profiling assay and in the NGS genotyping results obtained from the same primary DNA sample are compared. A strictly identical profile validates the NGS genotyping results.

The minimal number of SNPs to be analysed in order to validate NGS genotyping results depends on the number N of patients tested in the NGS assay and on the frequency of the two alleles of each SNP in the tested population of patients. For biallelic SNPs, the term "minor allele frequency (MAF)" refers to the frequency at which the less common allele (minor allele, or allele 2) occurs in a given population. Allele 1 refers to the most common allele in this population. MAF provides information to differentiate between common (MAF ≥1%) and rare variants (MAF <1%) in the population

Hence, the probability P that at least 2 patients among N patients present the same SNP profile is defined by the following formula:

$$P = 1 - (1 - F(p_1, \ldots, p_n))^{\frac{N(N-1)}{2}}$$

wherein "p" is the frequency of allele 1 (frequency of allele 2 is "1-p");

wherein "n" is the number of SNP tested;
wherein "$F(p_1, \ldots, p_n)$" is the probability that 2 patients have the same SNP profile for the n SNPs. $F(p_1, \ldots, p_n) = f(p_1)\ldots.f(p_n)$, wherein f(p) is the probability for two patients to have the same genotype for one SNP. $f(p) = (p^2)^2 + [2p(1-p)]^2 + [(1-p)^2]^2$, wherein $p^2$, $2p(1-p)$, and $(1-p)^2$ is the probability for one patient to have the 1/1 genotype, the 1/2 genotype, and the 2/2 genotype, respectively.

For example, the probabilities P that 2 patients present the same SNP profile with a combination of 12 SNPs (MAF = 0.4 for each SNP) according to the size of the series are as follows:

- 0.0007 for a series of 12 patients;
- 0.0030 for a series of 24 patients;
- 0.0121 for a series of 48 patients;
- 0.0481 for a series of 96 patients.

P should be as low as possible (to prevent necessity of further validation by sequencing). Preferably, SNPs will be selected so that P is ≤10%, preferentially ≤9%, more preferentially ≤5%, or even more preferentially ≤1%.

Depending on MAF of selected set of SNPs in the target population, the number of patients in the series, and the desired probability that 2 patients of the series present the same SNP profile, those skilled in the art will easily determine the minimal number of SNPs to be analysed to validate NGS genotyping results based on the above described formula.

For example, the minimal number of SNPs to be analysed in order to validate NGS genotyping results, with a probability to have 2 identical patients in a series of "N" patients to be less than 5%, may be as shown in Table 1 below. In a particular embodiment, the method according to the invention thus comprises the step of detecting at least n SNPs according to the number N of patients:

Table 1: Minimal number of SNPs to be analysed in order to validate NGS genotyping results.

| Number of patients (N) | Minimal number of SNPs (n; MAF=0.4) | P |
|---|---|---|
| 12 | 8 | 0.031751 |
| 24 | 10 | 0.019859 |

(continued)

| Number of patients (N) | Minimal number of SNPs (n; MAF=0.4) | P |
|---|---|---|
| 48 | 11 | 0.031115 |
| 96 | 12 | 0.048079 |
| 384 | 15 | 0.044535 |

To limit the minimal number of SNPs to be analysed to validate NGS genotyping results, selected SNPs should preferably not present significant linkage disequilibrium (preferably they do not present linkage disequilibrium) with each other and present a minor allele frequency (MAF) for the tested population comprised between 0.1 and 0.5.

Therefore, in a preferred embodiment, the SNPs according to the invention further have one or both of the following features:

v. they do not present significant linkage disequilibrium (LD) (preferably they do not present LD) between each other;

vi. they present a minor allele frequency (MAF) for a population comprised between 0.1 and 0.5, preferentially between 0.2 and 0.5, more preferentially between 0.25 and 0.5, even more preferentially between 0.275 and 0.5, even more preferentially between 0.3 and 0.5, even more preferentially between 0.325 and 0.5, even more preferentially between 0.35 and 0.5, even more preferentially between 0.375 and 0.5, more preferentially between 0.4 and 0.5.

Preferentially said SNPs according to the invention have the features v. and vi.

"Linkage disequilibrium" (also referred to as LD) is defined as the trend for alleles at nearby loci on haploid genomes to correlate in the population. Loci are said to be in linkage disequilibrium when the frequency of association of their different alleles is higher or lower than what would be expected if the loci were independent and associated randomly. For example, b and c, alleles at close loci B and C, are said to be in linkage disequilibrium if the "b c" haplotype (a haplotype is defined as a set of alleles on the same chromosomal segment) has a frequency which is statistically higher than f(b) x f(c) (expected frequency if the alleles segregate independently, where f(b) is the frequency of allele b, and f(c) that of allele c).

By "population" it is meant herein a group of individuals that is determined by geographic, temporal and/or genetic heritage criteria. For instance, European American and African American populations are defined by NHLBI Exome Sequencing Project (ESP) relying on patient data collected by clinicians (Auer et al., 2016); and Exome Aggregation Consortium (ExAC) performed principal component analysis (PCA) to distinguish the major axes of geographic ancestry and to identify population clusters corresponding to individuals of Finnish European, non-Finnish European, African, South Asian, East Asian, Latino ancestry (Lek et al., 2016).

For example, if a particular genetic element (e.g., an allele of a polymorphic marker, or a haplotype) occurs in a population at a frequency of 0.50 (50%) and another element occurs at a frequency of 0.50 (50%), then the predicted occurrence of a person's having both elements is 0.25 (25%), assuming a random distribution of the elements. However, if it is discovered that the two elements occur together at a frequency higher than 0.25, then the elements are said to be in linkage disequilibrium, since they tend to be inherited together at a higher rate than what their independent frequencies of occurrence (e.g., allele or haplotype frequencies) would predict.

Therefore, SNPs according to the inventions should preferably not present significant linkage disequilibrium (preferably they do not present LD) with each other in order to provide independent information from each other and to increase the informativeness of the SNP profiling assay.

Methods to conduct LD analysis and identify SNPs in (significant) LD can be carried out by the skilled person without undue experimentation by using well-known methods. Thus, the practitioner of ordinary skill in the art can easily identify SNPs in (significant) linkage disequilibrium.

Such markers are mapped and listed in public databases like Genome Variation Server (GVS, http://gvs.gs.washington.edu) as well known to the skilled person. Genomic LD maps have been generated across the genome, and such LD maps have been proposed to serve as framework for mapping disease-genes (Risch et al, 1996; Maniatis et al, 2002; Reich et al, 2001).

The two metrics most commonly used to measure LD are D' and r2 and can be written in terms of each other and allele frequencies. Both measures range from 0 (the two alleles are independent or in equilibrium) to 1 (the two alleles are completely dependent or in complete disequilibrium), but with different interpretation. D' is equal to 1 if at most two or three of the possible haplotypes defined by two markers are present, and <1 if all four possible haplotypes are present. r2 measures the statistical correlation between two markers and is equal to 1 if only two haplotypes are present. It is generally considered that significant LD is present when $r2\geq0.8$. In the context of the invention, any pair of selected SNPs preferably has a r2<0.8, preferably r2<0.75, r2<0.7, r2<0.65, r2<0.6, r2<0.55, r2<0.5, r2<0.45, r2<0.4, or r2<0.35. r2 values of two SNPs located in close parts of the genome (for instance in the same locus) may notably be found in Genome Variation Server (GVS, http://gvs.gs.washington.edu).

Another method to assess significant LD between two biallelic SNPs that are located in close regions of the genome (for instance in the same gene, or in two close loci) is based on the comparison of the MAF of the two SNPs. If the MAF of the two SNPs is the same or nearly the same (≤10% variation), it may be considered that the two SNPs are probably in significant LD. MAFs of SNPs are available to those skilled in the art in various databases, such as NHLBI Exome Sequencing Project (ESP) - Exome Variant Server (http://evs.gs.washington.edu/EVS/), Exome Aggregation Consortium - ExAC (http://exac.broadinstitute.org/), or Genome Aggregation Database - gnomAD (http://gnomad.broadinstitute.org/).

In a preferred embodiment, the SNPs according to the invention are located in housekeeping genes.

The term "housekeeping" gene refers to a group of genes that codes for proteins whose activities are essential for the maintenance of cell function. Accordingly, housekeeping gene are not likely to be related to disease, and are therefore reducing the risk of unsolicited finding, in contrast to Pengelly et al. (2013).

In the sense of the invention, the terms "combination of SNPs" and "set of SNPs" both indistinctly designate at least two different SNPs whose genotypes are determined in order to obtain a SNP profile.

In a preferred embodiment, combination of SNPs according to the invention comprises at least one of rs11702450; rs843345; rs1058018; rs8017; rs3738494; rs1065483; rs2839181; rs11059924; rs2075144; rs6795772; rs456261; rs1131620; rs2231926; rs352169 and rs3739160 (Table 2). Preferably, combination of SNPs according to the invention comprises at least 2, preferentially at least 8 SNPs, more preferentially at least 12 SNPs, and even more preferably 15 SNPs selected from rs11702450; rs843345; rs1058018; rs8017; rs3738494; rs1065483; rs2839181; rs11059924; rs2075144; rs6795772; rs456261; rs1131620; rs2231926; rs352169 and rs3739160.

Table 2: Selected SNPs

| SNP | Chromosome | Coordinate[1] | Ref[2] | Alt[3] | dbSNP ID[4] | Gene | Sense[5] | NM[6] | Nomenclature (HGVS)[7] |
|---|---|---|---|---|---|---|---|---|---|
| 1 | chr21 | 47703649 | G | A | rs11702450 | *MCM3AP* | AS[8] | NM_003906 | c.1323C>T |
| 2 | chr3 | 183906515 | T | C | rs843345 | *ABCF3* | S[9] | NM_018358 | c.837-34T>C |
| 3 | chr17 | 47000251 | C | T | rs1058018 | *UBE21* | S | NM_023079 | c.846C>T |
| 4 | chr16 | 2821573 | C | T | rs8017 | *ELOB*[10] | AS | NM_207013 | c.386G>A |
| 5 | chr1 | 43124859 | C | T | rs3738494 | *PPIH* | S | NM_006347 | c.132-40C>T |
| 6 | chr17 | 5284770 | G | A | rs1065483 | *RABEP1* | S | NM_004703 | c.2457G>A |
| 7 | chr21 | 47685939 | A | G | rs2839181 | *MCM3AP* | AS | NM_003906 | c.2931T>C |
| 8 | chr12 | 129293346 | C | T | rs11059924 | *SLC15A4* | AS | NM_145648 | c.1245G>A |
| 9 | chr19 | 46857286 | G | A | rs2075144 | *PPP5C* | S | NM_006247 | c.363+40G>A |
| 10 | chr3 | 49365269 | C | T | rs6795772 | *USP4* | AS | NM_003363 | c.230-20G>A |
| 11 | chr6 | 33258443 | G | A | rs456261 | *PFDN6* | S | NM_001265595 | c.261-50G>A |
| 12 | chr19 | 41117869 | A | G | rs1131620 | *LTBP4*[11] | S | NM_001042544 | c.2359A>G |
| 13 | chr3 | 73111809 | A | G | rs2231926 | *PPP4R2* | S | NM_174907 | c.420-1015A>G |
| 14 | chr3 | 52236762 | G | A | rs352169 | *ALAS1* | S | NM_000688 | c.427+12G>A |
| 15 | chr2 | 105654716 | C | T | rs3739160 | *MRPS9* | S | NM_182640 | c.135+31C>T |

[1] Human assembly GRCh37/hg19 coordinate.
[2] Reference: reference base at the position on the sense strand of the chromosome.
[3] Alternate: alternate base
[4] Single Nucleotide Polymorphism database (dbSNP), https://www.ncbi.nlm.nih.gov/projects/SNP/
[5] Sense of transcription
[6] RefSeq accession number for mRNA
[7] Conventional SNP nomenclature takes into account the direction of transcription of the gene and the RefSeq accession number for mRNA. If the gene is transcribed in sense, the SNP nomenclature uses the bases as indicated in the Ref and Alt columns. If the gene is transcribed in antisense, the SNP nomenclature uses the bases complementary to those listed in the Ref and Alt columns. HGVS = Human Genome Variation Society, http://www.hgvs.org/
[8] AS: antisense
[9] S: sense
[10] previously *TCEB2*
[11] associated to the phenotype 613177 according to OMIM, https://www.omim.org/

In a preferred embodiment, combination of SNPs according to the invention consists of all of rs11702450; rs843345; rs1058018; rs8017; rs3738494; rs1065483; rs2839181; rs11059924; rs2075144; rs6795772; rs456261; rs1131620; rs2231926; rs352169 and rs3739160. The use of these 15 SNPs allows in particular to validate NGS genotyping results in a series of 96 patients with a probability P that at least 2 patients present the same SNP profile (Table 3), according to the patients' origin and MAF of each SNP (detailed in Section 'EXAMPLES'), of:

Table 3: Probability P that at least 2 patients among 96 patients present the same SNP profile.

| Origin | P | MAFs data from |
|---|---|---|
| African | 0.003995 | ExAC[1] |
| East Asian | 0.035574 | ExAC |
| European (Finnish) | 0.002459 | ExAC |
| European (Non-Finnish) | 0.002129 | ExAC |
| Latino | 0.004724 | ExAC |
| South Asian | 0.006313 | ExAC |
| European American | 0.002174 | EVS[2] |
| African American | 0.003806 | EVS |

[1] Exome Aggregation Consortium (ExAC)
[2] NHLBI Exome Sequencing Project (ESP) - Exome Variant Server

These SNPs fulfill all criteria mentioned above for SNPs, i.e. they are not located in a repeated sequence of the genome; they are biallelic; the 60 bases flanking sequences at either side of the SNP site has a GC content <70% and an AT content <70%; they are not associated to a known pathology; they do not present significant linkage disequilibrium between each other; they present a minor allele frequency (MAF) between 0.39 and 0.5 for European American population, or between 0.21 and 0.5 for African American population, and they are located in housekeeping genes.

*Preferred method for independent SNP profiling assay*

No matter which set of SNPs is used, all of said SNPs according to the invention are detected by allele-specific multiplex PCR with a specific set of primers, wherein said specific primers have the following features:

I. no additional SNP of frequency >5% is present within the said specific primers, and no additional SNP of frequency >1% is present within the 10 bases of the 3' end of the said specific primers; and
II. their melting temperature is comprised between 62°C and 71 °C, preferentially between 63°C and 68°C, more preferentially between 64 and 66°C, even more preferentially about 65°C (+/-1 °C); and
III. they generate amplicons which do not contain any repeat, insertion or deletion frequent (>1%) polymorphism;

wherein said specific set of primers comprises for each SNP the following triplet of primers:

a) 2 primers ("sense strand primers"; Fig. 1) hybridizing, on the same DNA strand, specifically, at their 3' end, to the polymorphic nucleotide of alleles 1 and 2 of said SNP, respectively;
b) 1 primer specifically hybridizing to the opposite strand ("opposite strand primer").

Such a triplet may be subdivided into two pairs of primers, one for each allele (1 or 2) of the SNP, comprising each a sense strand primer and an opposite strand primer.

The absence of additional SNP within the primers sequences according to point I. above prevents allele drop-out (i.e. preferential amplification of one out of both alleles; hybridization of primer on allele containing additional SNP would be incomplete, thus weaker than the one on the other allele, resulting in preferential amplification of allele that does not contain the additional SNP, on which hybridization of primer is complete and strong).

The PCR efficiency is further improved by selecting primers with high melting temperature comprised between 62°C and 71°C, preferentially between 63°C and 68°C, more preferentially between 64 and 66°C, even more preferentially about 65°C (+/-1 °C), according to point II. above, which enhance annealing specificity of the all primer set and tend to equalize yields of PCR amplification for all SNPs.

Further, as described at point III. above, primers are also designed to generate amplicons which do not contain any repeat, insertion or deletion frequent (>1%) polymorphism, that could modify the expected amplicon size and thus jeopardize the discriminatory power of the method based on the detection of amplicons of different sizes.

By "multiplex PCR" or "allele-specific multiplex PCR" it is meant a molecular biology technique for amplification of multiple targets in a single PCR reaction. In an allele-specific multiplex PCR assay, more than one target sequence can be amplified by using multiple primers in the same reaction mixture.

The term "sense strand primer" refers to the primer designed to hybridize specifically, at its 3' end, to the polymorphic nucleotide of allele 1 or 2 of a particular SNP (Figure 1). The "opposite strand primer" is therefore the primer designed to hybridize specifically to the opposite strand of the DNA targeted sequence used to design the sense strand primer. The same opposite strand primer is used to amplify alleles 1 and 2. Hence, a pair of primers according to the invention consists in a sense strand primer and opposite strand primer adapted to specifically amplify the DNA sequence of the allele 1 or the allele 2 of a particular SNP of interest.

PCR methods, conditions and reagents are known in the art. Generally, PCR amplification is conducted in a PCR reaction mixture that includes a template nucleic acid molecule containing the sequence that is sought to be amplified, complementary primers designed to hybridize to particular target sites on the template, deoxyribonucleotide triphosphates (dNTPs), and a DNA polymerase, all combined in a suitable buffer that allows annealing of the primers to the template and provides conditions and any cofactors or ions necessary for the DNA polymerase to extend the primer to result in new DNA product, also call "amplicon" or PCR product.

Further, PCR methods consist in subjecting PCR reaction mixture to cycle of varying temperatures and for pre-determined times that allow for the steps of denaturation, annealing and elongation. Generally, the denaturation, annealing and elongation steps of the PCR cycle each occur at a different specific temperature and it is known in the art to conduct the PCR in a thermal cycler to achieve the required temperature for each step of the PCR cycle. Denaturation is typically performed at the highest temperature to melt any double stranded DNA (either template or amplified product formed in previous cycles), for example about 95°C if a heat resistant DNA polymerase such as *Taq* polymerase is used. The annealing step is performed at a temperature that allows for the primers to specifically hybridize to their complementary DNA strand target, and is typically chosen to facilitate specific annealing while reducing non-specific base pairing. Annealing temperature is chosen according to the melting temperature of the primers included in the PCR reaction mixture, which depends on the sequence of the primers. As used herein, the term "annealing temperature" refers to the temperature used during PCR to allow a primer to form specific base pairs with a complementary strand of DNA. Typically, the annealing temperature for a particular set of primers is chosen to be slightly below the average melting temperature, for example about 1°C, about 2°C, about 3°C or about 4°C below, preferentially 1°C below, although it may in some instances be equal to or slightly above the average melting temperature for the particular set of primers, especially for allele-specific multiplex PCR. In the context of the invention, primers are preferentially designed to have a melting temperature comprised between 62°C and 71°C, preferentially between 63°C and 68°C, more preferentially between 64 and 66°C, even more preferentially about 65°C (+/-1°C) and the annealing temperature is preferentially 65°C (+/-1°C). The selection of a high annealing temperature (about 65°C) and of primers with corresponding high melting temperature as defined above permits to limit or even prevent the formation of 3'dimers of primers with themselves, with the other primer of their pair, and with other primers of other pairs of the set of primers. Indeed, the energy of binding of such primers with high melting temperature for use at a high annealing temperature to their target is much lower (in general between -35 Kcal/mol to -60 Kcal/mol) than that of possible 3'dimers of primers (see values defined below). The elongation step is performed at a temperature suitable for the particular DNA polymerase enzyme used, to allow the DNA polymerase to synthesize amplified product, or amplicon.

The "melting temperature" of an oligonucleotide (or primer) is defined as the temperature at which 50% of that oligonucleotide are in duplex (double strand with its perfect complementary sequence) and the other 50% are single strand molecules.

In a particular embodiment, the specific primers of each pair consisting of a sense primer and an opposite primer intended for amplifying one allele of an SNP according to the invention further have at least one of the following features:

IV. they do not form dimer at their 3'end with themselves, nor with each other, whose binding energy is below -3.6 Kcal/mol, preferentially -1.9 Kcal/mol.

Although not mandatory, the binding energy of 3'end dimers formed between primers intended for distinct SNPs should preferably be at least -25 Kcal/mol, preferably at least -20 Kcal/mol, even more preferably at least -15 Kcal/mol. If possible (depending on the number of SNPs present in the SNP profiling assay and constraints deriving from this number), the binding energy of most (at least 50%, at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, or even 100%) 3'end dimers formed between primers intended for distinct SNPs should be at least -10 Kcal/mol, preferably at least -9 Kcal/mol, at least -8 Kcal/mol, at least -7 Kcal/mol, at least -6 Kcal/mol, at least -5 Kcal/mol, or even at least -4 Kcal/mol or at least -3.6 Kcal/mol.

V. they do not hybridize to the genome unspecifically;

VI. they generate amplicons with a size comprised between 90 and 500 base pairs.

In particular, features IV. and V. prevent synthesis of unspecific PCR products and allow to increase primers availability to enhance efficiency of the PCR amplification, while feature VI. tends to equalize the yield of PCR, allows to shorten the PCR elongation step, and therefore maintains the efficiency of the polymerase through the PCR cycles.

Hence, selected pair of primers should not be capable of forming dimers or hybridize to the genome unspecifically, since this can interfere with primer annealing to a target locus and thus reduce efficiency of the amplification.

In a preferred embodiment, the specific pair of primers according to the invention have all of the above features I. to VI.

In a preferred embodiment of any set of primers described above (fulfilling primer criteria I. to III. and optionally at least one or all of primer criteria IV. to VI.), the 2 sense strand primers according to the invention comprise at least one base at the 3' end which is a Locked Nucleic Acid (LNA) base (criteria VII).

As used herein, the term "locked nucleic acid(s)", or "LNA", refers to type of nucleic acid analog that contains a 2'-O, 4'-C methylene bridge. LNA nucleotides can be mixed with DNA residues in the primer whenever desired. The bridge -locked in the 3'-endo conformation-restricts the flexibility of the ribofuranose ring and locks the structure into a rigid bicyclic formation. This significantly increases the hybridization properties (melting temperature) of primers. In particular, LNA oligonucleotides are used to increase the sensitivity and specificity of the PCR. Hence, it is included herein any modified nucleotide that allows to also increase the sensitivity and specificity of the amplification of the PCR.

In a preferred embodiment of any set of primers described above (fulfilling primer criteria I. to III. and optionally at least one or all of primer criteria IV. to VI., and optionally criteria VII.), said opposite strand primers or sense strand primers, preferentially opposite strand primers, according to the invention have an additional GTTTCTT sequence added to their 5' end (criteria VIII.). Preferentially, primers comprising said additional GTTTCTT sequence do not form dimer at their 3'end with themselves or with both sense strand primers of said pair of primers, or preferentially with other primers of said set whose binding energy is below -3.6 Kcal/mol. Additional GTTTCTT sequence added to the 5' end of the opposite strand primers allow to stabilize and to reduce the "plus-A artifact" during PCR (Brownstein et al., 1996). "Plus-A artifact" results from the tendency of the Taq polymerase to add a non-templated nucleotide (usually a A) to the 3' end of the double stranded DNA.

In one embodiment, in addition to primer criteria I. to III. defined above, the specific primers of each pair consisting of a sense primer and an opposite primer intended for amplifying one allele of an SNP according to the invention further comprise at least one of the following features:

IV. they do not form dimer at their 3'end with themselves, nor with each other, whose binding energy is below -3.6 Kcal/mol, preferentially -1.9 Kcal/mol.
Although not mandatory, the binding energy of 3'end dimers formed between primers intended for distinct SNPs should preferably be at least -25 Kcal/mol, preferably at least -20 Kcal/mol, even more preferably at least -15 Kcal/mol. If possible (depending on the number of SNPs present in the SNP profiling assay and constraints deriving from this number), the binding energy of most (at least 50%, at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, or even 100%) 3'end dimers formed between primers intended for distinct SNPs should be at least -10 Kcal/mol, preferably at least -9 Kcal/mol, at least -8 Kcal/mol, at least -7 Kcal/mol, at least -6 Kcal/mol, at least -5 Kcal/mol, or even at least -4 Kcal/mol or at least -3.6 Kcal/mol.
V. they do not hybridize to the genome unspecifically;
VI. they generate amplicons with a size comprised between 90 and 500 base pairs;
VII sense strand primers comprise at least one Locked Nucleic Acid (LNA) base at the 3' end; and
VIII opposite strand primers or sense strand primers, preferentially opposite strand primers, have an additional GTTTCTT sequence added to their 5' end.

Preferentially, in addition to primer criteria I. to III. defined above, the specific pair of primers according to the invention further comprise all of the features IV. to VIII.

In a preferred embodiment, the pairs of primers intended to amplify one allele of an SNP (fulfilling primer criteria I. to III. and optionally at least one or all of primer criteria IV. to VI., and optionally VII. and/or VIII.) according to the invention are further designed to generate amplicons of different sizes, wherein:

IX. the sizes of amplicons related to the allele 1 and the allele 2 of $SNP^n$ differ by 2 to 5 base pairs, preferentially 3 base pairs; and
X. the sizes of amplicons related to the allele 2 of $SNP^n$ and the allele 1 of $SNP^{n+1}$ differ by 2 to 20 base pairs, preferentially 2 to 10 base pairs, more preferentially 3 to 8 base pairs, even more preferentially 4 to 6 base pairs, preferentially 5 base pairs; and
XI. said difference between the sizes of amplicons of allele 1 and allele 2 of each SNP is generated by adding bases to the 5'end of the sense strand primer hybridizing with allele 1 or 2 of the SNP, preferentially allele 2 of the SNP.

Sense strand primers designed to detect allele 1 and the allele 2 of $SNP^n$ according to point IX. differ by 2 to 5 bases (see above for preferred ranges and values), preferentially 3 bases, to allow on one side to efficiently discriminate amplicons of allele 1 and 2 according to their sizes, and on the other side to limit the difference in melting temperature between the primers. Limitation of the difference in melting temperature is important to optimize annealing temperature, in order to have equivalent PCR yield for both alleles.

Further, sizes of amplicons related to the allele 2 of $SNP^n$ and the allele 1 of $SNP^{n+1}$differ by 2 to 20 base pairs (see above for preferred ranges and values), to allow on one side to efficiently discriminate amplicons from allele 2 of $SNP^n$ and amplicons from allele 1 of $SNP^{n+1}$ according to their sizes, and on the other side to limit the size of all the amplicons between 90 and 500 bases. Limitation of the size of all the amplicons between 90 and 500 bases is important to obtain similar yield for each PCR product, and to shorten PCR elongation step, which enhance PCR efficiency, and time to result.

In a preferred embodiment, SNPs according to the invention are detected by determining the size of said amplicons generated by allele-specific multiplex PCR, preferably by method for separation of DNA based on size, such as capillary electrophoresis. Such method for separation of DNA based on size are well known in the art. Based on the size of the amplicons detected, SNPs genotype can be determined and SNPs profile of the patient can be established.

In a particular embodiment, the sense strand primers or the opposite strand primers according to the invention are labeled with a fluorochrome, such as 6-FAM. It should be noted that, when the sense or opposite primers have a GTTTCTT sequence at their 5' end, the fluorochrome is attached to primer not comprising the GTTTCTT sequence at their 5' end, i.e. the sense strand primers are labeled with a fluorochrome if the opposite strand primers have the GTTTCTT sequence at their 5' end, while the opposite strand primers are labeled with a fluorochrome if the sense strand primers have the GTTTCTT sequence at their 5' end). This method is particularly suited for detection of SNPs base on size of DNA amplicons separated by capillary electrophoresis. Advantageously, the fluorochrome of the invention can be identified or distinguished from other labels, and allow discrimination of different labeled amplicons. Examples of fluorochrome or fluorescent label are 6-FAM, HEX, TET or NED dye. Differentially labeled primers allow to distinguish different PCR amplification products (multi-color multiplex PCR) even if their length (size) are approximately the same.

In a particular embodiment, the combination of SNPs according to the invention comprises at least one, preferentially at least 2, preferentially at least 8, more preferentially at least 12, even more preferentially all of rs11702450; rs843345; rs1058018; rs8017; rs3738494; rs1065483; rs2839181; rs11059924; rs2075144; rs6795772; rs456261; rs1131620; rs2231926; rs352169 and rs3739160, and the following primers are used for each of the SNPs:

Table 4. Primers sequences and labels

| SNP | SEQ ID NO | NAME | PRIMER SEQUENCE AND LABEL |
|---|---|---|---|
| rs11702450 | SEQ ID NO 1 | MCM3AP_1323CL_F_Label | [LABEL]CACAGCCATCCAGTGCAAGAA{C} |
| | SEQ ID NO 2 | MCM3AP_1323TL_F_Label | [LABEL]CAACACAGCCATCCAGTGCAAGAA{T} |
| | SEQ ID NO 3 | MCM3AP_ex2_q7_R | GTTTCTTAAGATGCGCTGCACTTTAGCAA |
| rs843345 | SEQ ID NO 4 | ABCF3_837-34TL_R_Label | [LABEL]AGAAACAGCAATTGGCCTAAGC{A} |
| | SEQ ID NO 5 | ABCF3_837-34CL_R_Labet | [LABEL]ATGAGAAACAGCAATTGGCCTAAGC{G} |
| | SEQ ID NO 6 | ABCF3_q7_F | GTTTCTTATTCTCTTCCTCTTCCAGCCACA |
| rs1058018 | SEQ ID NO 7 | UBE2Z_846CL_R_Labet | [LABEL]GATCTTTGCAGGCCACCTC{G} |
| | SEQ ID NO 8 | UBE2Z_846TL_R_Label | [LABEL]GATGATCTTTGCAGGCCACCTC{A} |
| | SEQ ID NO 9 | UBE2Z_q7_F | GTTTCTTTGACCTGTACCCCTGGGTTTCT |

(continued)

| SNP | SEQ ID NO | NAME | PRIMER SEQUENCE AND LABEL |
|---|---|---|---|
| rs8017 | SEQ ID NO 10 | TCEB2_386GL_R_Labet | [LABEL]GGCTCCAGCTTGTGTTTCTG{C} |
| | SEQ ID NO 11 | TCEB2_386AL_R_Label | [LABEL]TTGGGCTCCAGCTTGTGTTTCTG{T} |
| | SEQ ID NO 12 | TCEB2_q7_F | GTTTCTTCCAGCCTCAGGGACAAGAGATT |
| rs3738494 | SEQ ID NO 13 | PPIH-132-40CL_F_Label | [LABEL]GAGGCGCTCACGACTGTGA{C} |
| | SEQ ID NO 14 | PPIH_132-40TL_F_Label | [LABEL]CAAGAGGCGCTCACGACTGTGA{T} |
| | SEQ ID NO 15 | PPIH_q7_R | GTTTCTTACCCCTCTGGAGCAGGCAA |
| rs1065483 | SEQID NO 16 | RABEP1_2457GL2_F_Label | [LABEL]GATGTCAGTGAGCAAGTCCAGA{GG} |
| | SEQ ID NO 17 | RABEP1_2457AL-F_Label | [LABEL]AGAGATGTCAGTGAGCAAGTCCAGAG{A} |
| | SEQ ID NO 18 | RABEP1_q7_R | GTTTCTTCAGTGGTCAAGTCAGGGATCGG |
| rs2839181 | SEQ ID NO 19 | MCM3AP_2931TL_R_Label | [LABEL]TTGAAGCTGCACACAGGGGT{A} |
| | SEQ ID NO 20 | MCM3AP_2931CL_R_Label | [LABEL]TCATTGAAGCTGCACACAGGGGT{G} |
| | SEQ ID NO 21 | MCM3AP_q7_F | GTTTCTTGTCTGCATTCCTGGAACCAGAG |
| rs11059924 | SEQ ID NO 22 | SLC15A4_1245GL_F_Label | [LABEL]GCATGTTCTTTGTCATGTGCTC{G} |
| | SEQ ID NO 23 | SLC15A4_1245AL_F_Label | [LABEL]AACGCATGTTCTTTGTCATGTGCTC{A} |
| | SEQ ID NO 24 | SLC15A4_q7_R | GTTTCTTTTTACAGACATGCACTTCCTGAACAAC |
| rs2075144 | SEQ ID NO 25 | PPP5C_363+40GL_R_Label | [LABEL]GCCCAGCCCTCAGTATCTG{C} |
| | SEQ ID NO 26 | PPP5C_363+40AL_R_Label | [LABEL]TTCGCCCAGCCCTCAGTATCTG{T} |
| | SEQ ID NO 27 | PPP5C_q7_F | GTTTCTTCCATTGAGCTGGACAAGAAGTACATC |
| rs6795772 | SEQ ID NO 28 | USP4_230-20GL_F_Label | [LABEL]TCTGGGGTAAAGAGCAGTGACTTAT{G} |
| | SEQ ID NO 29 | USP4_230-20AL_F_Label | [LABEL]ACATCTGGGGTAAAGAGCAGTGACTTAT{A} |
| | SEQ ID NO 30 | USP4_q7_R | GTTTCTTCGATGGGTTGCTGGCCTTCTA |

(continued)

| SNP | SEQ ID NO | NAME | PRIMER SEQUENCE AND LABEL |
|---|---|---|---|
| rs456261 | SEQ ID NO 31 | PFDN6_261-50GL_R_Label | [LABEL]CAAGCAGAAAGGGAGAAATTAGTAGGACT{C} |
| | SEQ ID NO 32 | PFDN6_261-50AL_R_Label | [LABEL] TGACAAGCAGAAAGGGAGAAATTAGTAGGACT{T} |
| | SEQ ID NO 33 | PFDN6_q7_F | GTTTCTTAACCATTGCAGAACAGCTCTCCAT |
| rs1131620 | SEQ ID NO 34 | LTBP4_2359AL_R_Label | [LABEL]CGCACTCGGAGCCAGCAG{T} |
| | SEQ ID NO 35 | LTBP4_2359GL2_R_Label | [LABEL]TGACGCACTCGGAGCCAGCA{GC} |
| | SEQ ID NO 36 | LTBP4_q7_F | GTTTCTTTGATGGCCATGGGAATGGAT |
| rs2231926 | SEQ ID NO 37 | PPP4R2_420-1015AL_R_ Label | [LABEL]TTATCACTTGATCCAGCCGCAA{T} |
| | SEQ ID NO 38 | PPP4R2_420-1015GL2_R_ Label | [LABEL]CAGTTATCACTTGATCCAGCCGCA{AC} |
| | SEQ ID NO 39 | PPP4R2_q7_F | GTTTCTTGATGGGTTACACCAGGCATTACTGA |
| rs352169 | SEQ ID NO 40 | ALAS1_427+12GL_F_Label | [LABEL]CCGTGAGGAAAGGTAAGAGATGA{G} |
| | SEQ ID NO 41 | ALAS1_427+12AL_F_Label | [LABEL]ACTCCGTGAGGAAAGGTAAGAGATGA{A} |
| | SEQ ID NO 42 | ALAS1_q7_R | GTTTCTTCGCACCAGAAAGAAAGTCCCA |
| rs3739160 | SEQ ID NO 43 | MRPS9_135+31CL_F_Label | [LABEL]GGAAGACTGGAAGCGGCTTA{C} |
| | SEQ ID NO 44 | MRPS9_135+31TL_F_Label | [LABEL]CATGGAAGACTGGAAGCGGCTTA{T} |
| | SEQ ID NO 45 | MRPS9_q7_R | GTTTCTTAGGTCGCTCCACTTCTACCTTCA |

wherein bases in braces are LNA modified bases; [LABEL] is the 5' labelling modification of the primer. Said labelling modification may be selected from 5' fluorescent modifications, 5' radioactive modifications, 5' luminescent modifications, and any other appropriate 5' modification permitting detection of the amplification product. Preferably, said labelling modification is a 5' fluorescent modification by any suitable fluorescent label, including 6FAM (6-carboxyfluorescein), TET, VIC, HEX, NED, PET, JOE, ROX, TAMRA, Cy® dyes, Alexa Fluor® Dyes, ATTO-TEC Dyes, Dragonfly Orange™, Texas Red®, Yakima Yellow®, Fluorescein. Preferably the 5' fluorescent modification is a 5' 6FAM modification.

In Table 4 above, for each SNP, the first primer is the sense strand primer specific for allele 1; the second is the sense strand primer specific for allele 2; the third is the opposite strand primer. Bases in braces are LNA modified bases; [LABEL] is the 5' labelling modification of the primer; bases in bold characters are the three bases added at the 5' end of the sense strand primer specific for allele 2.

In a preferred embodiment, the primers are labelled with a fluorescent modification in their 5' end.

Therefore, in a preferred embodiment, the combination of SNPs according to the invention comprises at least one, preferentially at least 2, preferentially at least 8, more preferentially at least 12, even more preferentially all of rs11702450; rs843345; rs1058018; rs8017; rs3738494; rs1065483; rs2839181; rs11059924; rs2075144; rs6795772; rs456261; rs1131620; rs2231926; rs352169 and rs3739160, and the following primers are used for each of the SNPs:

Table 5. Primers sequences labelled with fluorescence

| SNP | SEQ ID NO | NAME | PRIMER SEQUENCE AND LABEL (6FAM) |
|---|---|---|---|
| rs11702450 | SEQ ID NO 1 | MCM3AP_1323CL_F_Fam | [6FAM]CACAGCCATCCAGTGCAAGAA{C} |
| | SEQ ID NO 2 | MCM3AP_1323TL_F_Fam | [6FAM]CAACACAGCCATCCAGTGCAAGAA{T} |
| | SEQ ID NO 3 | MCM3AP_ex2_q7_R | GTTTCTTAAGATGCGCTGCACTTTAGCAA |
| rs843345 | SEQ ID NO 4 | ABCF3_837-34TL_R_Fam | [6FAM]AGAAACAGCAATTGGCCTAAGC{A} |
| | SEQ ID NO 5 | ABCF3_837-34CL_R_Fam | [6FAM]ATGAGAAACAGCAATTGGCCTAAGC{G} |
| | SEQ ID NO 6 | ABCF3_q7_F | GTTTCTTATTCTCTTCCTCTTCCAGCCACA |
| rs1058018 | SEQ ID NO 7 | UBE2Z_846CL_R_Fam | [6FAM]GATCTTTGCAGGCCACCTC{G} |
| | SEQ ID NO 8 | UBE27_846TL_R_Fam | [6FAM]GATGATCTTTGCAGGCCACCTC{A} |
| | SEQ ID NO 9 | UBE2Z_q7_F | GTTTCTTTGACCTGTACCCCTGGGTTTCT |
| rs8017 | SEQ ID NO 10 | TCEB2_386GL_R_Fam | [6FAM]GGCTCCAGCTTGTGTTTCTG{C} |
| | SEQ ID NO 11 | TCEB2_386AL_R_Fam | [6FAM]TTGGGCTCCAGCTTGTGTTTCTG{T} |
| | SEQ ID NO 12 | TCEB2_q7_F | GTTTCTTCCAGCCTCAGGGACAAGAGATT |

(continued)

| SNP | SEQ ID NO | NAME | PRIMER SEQUENCE AND LABEL (6FAM) |
|---|---|---|---|
| rs3738494 | SEQ ID NO 13 | PPIH_132-40CL_F_Fam | [6FAM]GAGGCGCTCACGACTGTGA{C} |
| | SEQ ID NO 14 | PPIH_132-40TL-F-Fam | [6FAM]CAAGAGGCGCTCACGACTGTGA{T} |
| | SEQ ID NO 15 | PPIH_q7_R | GTTTCTTACCCCTCTGGAGCAGGCAA |
| rs1065483 | SEQ ID NO 16 | RABEP1_2457GL2_F_Fam | [6FAM]GATGTCAGTGAGCAAGTCCAGA{GG} |
| | SEQ ID NO 17 | RABEP1_2457AL_F_Fam | [6FAM]AGAGATGTCAGTGAGCAAGTCCAGAG{A} |
| | SEQ ID NO 18 | RABEP1_q7_R | GTTTCTTCAGTGGTCAAGTCAGGGATCGG |
| rs2839181 | SEQ ID NO 19 | MCM3AP_2931TL_R_Fam | [6FAM]TTGAAGCTGCACACAGGGGT{A} |
| | SEQ ID NO 20 | MCM3AP_2931CL_R_Fam | [6FAM]TCATTGAAGCTGCACACAGGGGT{G} |
| | SEQ ID NO 21 | MCM3AP_q7_F | GTTTCTTGTCTGCATTCCTGGAACCAGAG |

(continued)

| SNP | SEQ ID NO | NAME | PRIMER SEQUENCE AND LABEL (6FAM) |
|---|---|---|---|
| rs11059924 | SEQ ID NO 22 | SLC15A4_1245GL_F_Fam | [6FAM]GCATGTTCTTTGTCATGTGCTC{G} |
| | SEQ ID NO 23 | SLC15A4_1245AL_F_Fam | [6FAM]AACGCATGTTCTTTGTCATGTGCTC{A} |
| | SEQ ID NO 24 | SLC15A4_q7_R | GTTTCTTTTTACAGACATGCACTTCCTGAACAAC |
| rs2075144 | SEQ ID NO 25 | PPP5C_363+40GL_R_Fam | [6FAM]GCCCAGCCCTCAGTATCTG{C} |
| | SEQ ID NO 26 | PPP5C_363+40AL_R_Fam | [6FAM]TTCGCCCAGCCCTCAGTATCTG{T} |
| | SEQ ID NO 27 | PPP5C_q7_F | GTTTCTTCCATTGAGCTGGACAAGAAGTACATC |
| rs6795772 | SEQ ID NO 28 | USP4_230-20GL_F_Fam | [6FAM]TCTGGGGTAAAGAGCAGTGACTTAT{G} |
| | SEQ ID NO 29 | USP4_230-20AL_F_Fam | [6FAM]ACATCTGGGGTAAAGAGCAGTGACTTAT{A} |
| | SEQ ID NO 30 | USP4_q7_R | GTTTCTTCGATGGGTTGCTGGCCTTCTA |

(continued)

| SNP | SEQ ID NO | NAME | PRIMER SEQUENCE AND LABEL (6FAM) |
|---|---|---|---|
| rs456261 | SEQ ID NO 31 | PFDN6_261-50GL_R_Fam | [6FAM]CAAGCAGAAAGGGAGAAATTAGTAGGACT {C} |
| | SEQ ID NO 32 | PFDN6_261-50AL_R_Fam | [6FAM] TGACAAGCAGAAAGGGAGAAATTAGTAGGACT{T} |
| | SEQ ID NO 33 | PFDN6_q7_F | GTTTCTTAACCATTGCAGAACAGCTCTCCAT |
| rs1131620 | SEQ ID NO 34 | LTBP4_2359AL_R_Fam | [6FAM]CGCACTCGGAGCCAGCAG{T} |
| | SEQ ID NO 35 | LTBP4_2359GL2_R_Fam | [6FAM]TGACGCACTCGGAGCCAGCA{GC} |
| | SEQ ID NO 36 | LTBP4_q7_F | GTTTCTTTGATGGCCATGGGAATGGAT |
| rs2231926 | SEQ ID NO 37 | PPP4R2_420-1015AL_R_Fam | [6FAM]TTATCACTTGATCCAGCCGCAA{T} |
| | SEQ ID NO 38 | PPP4R2_420-1015GL2_R_Fam | [6FAM]CAGTTATCACTTGATCCAGCCGCA{AC} |
| | SEQ ID NO 39 | PPP4R2_q7_F | GTTTCTTGATGGGTTACACCAGGCATTACTGA |

(continued)

| SNP | SEQ ID NO | NAME | PRIMER SEQUENCE AND LABEL (6FAM) |
|---|---|---|---|
| rs352169 | SEQ ID NO 40 | ALAS1_427+12GL_F_Fam | [6FAM]CCGTGAGGAAAGGTAAGAGATGA{G} |
| | SEQ ID NO 41 | ALAS1_427+12AL_F_Fam | [6FAM]ACTCCGTGAGGAAAGGTAAGAGATGA{A} |
| | SEQ ID NO 42 | ALAS1_q7_R | GTTTCTTCGCACCAGAAAGAAAGTCCCA |
| rs3739160 | SEQ ID NO 43 | MRPS9_135+31CL_F_Fam | [6FAM]GGAAGACTGGAAGCGGCTTA{C} |
| | SEQ ID NO 44 | MRPS9_135+31TL_F_Fam | [6FAM]CATGGAAGACTGGAAGCGGCTTA{T} |
| | SEQ ID NO 45 | MRPS9_q7_R | GTTTCTTAGGTCGCTCCACTTCTACCTTCA |

wherein bases in braces are LNA modified bases; [6FAM] is the 5' fluorescent modification of the primer.

In Table 5 above, for each SNP, the first primer is the sense strand primer specific for allele 1; the second is the sense strand primer specific for allele 2; the third is the opposite strand primer. Bases in braces are LNA modified bases; [6FAM] is the 5' fluorescent modification of the primer; bases in bold characters are the three bases added at the 5' end of the sense strand primer specific for allele 2.

In another embodiment of the invention, the combination of SNPs according to the invention consists in all of rs11702450; rs843345; rs1058018; rs8017; rs3738494; rs1065483; rs2839181; rs11059924; rs2075144; rs6795772; rs456261; rs1131620; rs2231926; rs352169 and rs3739160, and the primers identified in Table 4 or Table 5 are used for each of the SNPs, respectively.

In one embodiment, SNP profiling assay in said step b) according to the invention is automated with a software recognizing the said labeled multiplex PCR products.

By "software recognizing the said multiplex labeled PCR products" it is referred herein to a software calculating size of each amplicon obtained by the method of the invention and attributing to each of them the corresponding SNP allele, according to their size or fluorescence, preferentially their size.

In one embodiment, the NGS method which results are validated using the method according to the invention is target capture NGS or amplicon NGS.

The term "target capture NGS" refers to NGS which is only perform on genomic regions of interest, which have been previously captured (or isolated) from a sample library. It is therefore important when using the target capture NGS method to choose the genomic regions of interest. Hence, when using capture NGS predefined manufacturer commercial kits, SNP genotyping is thus not immediately possible, and the SNP probes must be added to the kit by the manufacturer. And, when using capture NGS custom kits, genotyping of the SNPs is achieved by requesting to the manufacturer to add the SNP probes in a new version of the custom kit.

The term "amplicon NGS" refers to NGS which is only perform on genomic regions of interest which have been amplified from a DNA sample using primers designed to amplify regions of interest. For the NGS technique by amplicons using commercial kits predefined by the manufacturer, SNP genotyping is thus not immediately possible. Hence, in order to use amplicons NGS, the primers required for the amplification of regions surrounding the SNPs of interest, would have to be designed and added to the existing kit.

Another object of the present invention is a kit for detection of a combination of at least 8 SNPs in a method according to the invention as described above, comprising primers as defined above, said kit preferably further comprising:

- PCR multiplex reagents; and/or
- NGS oligonucleotide probes or primers designed to capture or amplify sequences comprising said at least 8 SNPs.

PCR multiplex reagents according to the invention may include, but are not limited to, DNA polymerase, dNTPs, buffer, any cofactors or ions necessary for the DNA polymerase to amplify the targeted sequence (e.g. QIAGEN Multiplex PCR Kit; Thermo Scientific Phusion™ U Multiplex PCR Master Mix; NEB Multiplex PCR 5X Master Mix). The use of Taq DNA polymerases and/or master mixes designed for simultaneous amplification of multiple targets in a single tube may reduce the need for PCR optimization.

Another object of the present invention is the use of the kit according to the invention in a method to validate NGS genotyping results of a panel of genes tested in series of at least 2 patients, according to the invention.

Another object of the present invention is a method for detecting polymorphisms in the DNA of a patients, comprising performing, preferentially in parallel, the two following steps:

a) detecting polymorphisms by NGS assay, and
b) validating NGS genotyping results using the above described method according to the invention.

The following examples merely intend to illustrate the present invention.

## EXAMPLES

### Example 1: Development of an allele-specific multiplex PCR SNP profiling assay for validation of target NGS genotyping results

In order to validate NGS genotyping results by comparing SNP profiles obtained by NGS assay and, independently, by another method, we designed an allele-specific multiplex PCR SNP profiling assay.

This SNP profiling assay has a high discrimination power, as the risk for two samples of a series of 96 patients to have the same SNP profile is less than 5%, whatever the origin of patient.

***Methods***

**SNP selection**

SNPs were selected according to the following criteria:

1. they are located in housekeeping gene (Eisenberg et al, 2003; Zhu et al, 2008);
2. they are not associated to a known pathology, i.e. they are not associated with Online Mendelian Inheritance in Man (OMIM) record;
3. their Minor Allele Frequency (MAF), as reported in NHLBI Exome Sequencing Project (ESP) - Exome Variant Server (http://evs.gs.washington.edu/EVS/), is between 0.39 and 0.5 in European Americans and between 0.21 and 0.5 in African Americans;
4. they are biallelic;
5. they do not present linkage disequilibrium between each other, i.e. they are preferentially not located in the same gene, and if located in the same gene, their MAF are significantly different;
6. they not located in a repeated sequence of the genome (tested by Repeat Masker http://www.repeatmasker.org. thru University of California Santa Cruz (UCSC) interface https://genome.ucsc.edu/);
7. the 60 bases flanking sequences at either side of the SNP site has a GC content < 70% and an AT content <70%.

**Primer design for allele-specific PCR**

Three primers were designed for each SNP: two sense strand primers hybridizing, on the same DNA strand, specifically, at their 3' end, to the polymorphic nucleotide of alleles 1 and 2 of the SNP; one opposite strand primer hybridizing to the opposite strand. Primers were designed according to the following criteria:

1. no additional SNP of frequency >5% is present within the primer, and no additional SNP of frequency >1% is present within the 10 bases of the 3' end of primer;
2. the melting temperature of the primer specific for allele 1 and complementary primer is preferentially about 65°C (+/-1 °C);
3. primer specific for allele 2 differs from primer specific for allele 1 by the base at its 3' end and by the addition of 3 bases at the 5' end of the primer (the sizes of amplicons related to allele 1 and allele 2 of $SNP^n$ will then differ by 3 base pairs);
4. the opposite strand primer has an additional GTTTCTT sequence added at its 5' end in order to stabilize and to reduce the "plus-A artifact" during PCR (Brownstein et al., 1996);
5. the three primers designed for one SNP do not form dimer at their 3'end with themselves or with each other, whose binding energy is below -3.6 Kcal/mol, preferentially -1.9 Kcal/mol;
6. they generate amplicons which do not contain any repeat, insertion or deletion frequent (>1%) polymorphism;
7. they do not hybridize significantly to the genome unspecifically (tested by Primer Blast https://www.ncbi.nlm.nih.gov/tools/primer-blast/);
8. they generate amplicons with a size comprised between 100 and 250 base pairs;
9. the sizes of amplicons related to the allele 2 of $SNP^n$ and the allele 1 of $SNP^{n+1}$ differ by 5 base pairs.

In order to increase the specificity of sense strand primers, one or two bases at their 3' end is a Locked Nucleic Acid (LNA) base. Sense strand primers are labeled at their 5' end by a 6FAM fluorescent dye.

Sense strand primers were synthetized and purified by HLPC by Eurogentec (www.eurogentec.com). Opposite strand primers were synthetized and purified by HLPC by Sigma Aldrich (www.sigmaaldrich.com).

**Allele-specific multiplex PCR**

Allele-specific multiplex PCR was performed with QIAGEN Multiplex PCR Kit (QIAGEN, Hilden, Germany). Several annealing temperatures, concentrations of primer, concentrations of 5x Q-Solution were tested in order to optimize the yield of PCR for each allele of each SNP.

Seven control DNA samples were selected in order to test each genotype (homozygous for allele 1, heterozygous for allele 1 and 2, and homozygous for allele 2) for each SNP.

PCR were performed using either Icycler (Bio-Rad, Hercules, California, USA) or GeneAmp® 9700 (Applied Biosystems, Waltham, Massachusetts, USA) PCR Thermal Cyclers.

PCR products were subjected to capillary electrophoresis using ABI PRISM 3730 DNA analyzer (Life Technologies). Raw data were analyzed by GeneMapper™ Software 5 software (Applied Biosystems).

**SNP genotyping by NGS assay**

The manufacturer of NGS capture custom kit (Roche NimbleGen Inc., Madison, Wisconsin, USA) was requested to add the probes corresponding to the selected SNP in a new version of the custom kit according to the coordinates of the regions of interest, i.e. SNP +/-100 bases.

NGS capture assay were performed according to the manufacturer instructions using a MiSeq System Illumina sequencing instrument (Illumina Inc., San Diego, California, USA). Bioinformatic analysis of data was performed by Genodiag (Genosplice, Paris, France).

**Test of stability of primer mix and mix-PCR**

Thirteen aliquots of mixes containing either only the primers (primer mix) or all the PCR reagents and primers (mix-PCR) were frozen at -20°C. One aliquot of each mix was used every month to perform PCR with the above mentioned selected DNA samples in order to evaluate the stability of mixes over one year.

**Robustness of SNP profiling assay**

Four variables have been considered and tested.

*PCR thermal cyclers.* The SNP profiling assay was performed for same selected samples on ten different PCR thermal cyclers: 8 Icycler and 2 GeneAmp® 9700 PCR Thermal Cyclers. *DNA quantity.* The SNP profiling assay was performed with 10, 25, 50, 100, 200, and 400 ng of DNA.

*Extraction method.* DNA samples obtained from saline extraction using standard procedure or from QIAsymphony SP instrument (QIAGEN, Hilden, Germany) were tested.

*Volume of mix-PCR.* Once the mix-PCR has demonstrated a perfect stability over a period of twelve months, the SNP profiling assay was performed with 49μl, 24μl, 14μl, or 9μl of mix-PCR mixed with one μl of primary DNA sample. For these experiments, primary DNA sample concentration was up to ≈ 800 ng/μl for DNA samples obtained from standard saline extraction method, standard phenol-chloroform extraction method, or FlexiGene DNA Kit (QIAGEN, Hilden, Germany); primary DNA sample concentration ranges from ≈ 100 to ≈ 350 ng/μl for DNA samples obtained from QIAsymphony SP instrument and from ≈ 100 to ≈ 200 ng/μl for DNA samples obtained from EZ1 DNA Tissue Kit or EZ1 DNA Blood 350 μl Kit. Ten ng of each DNA sample were also tested in parallel.

*Results*

**Allele-specific multiplex PCR SNP profiling assay**

The selected SNP and their frequencies according to Exome Variant Server, http://evs.gs.washington.edu/EVS/ and Exome Aggregation Consortium (ExAC), http://exac.broadinstitute.org/ are listed in Table 6.

The MAF between 0.39 and 0.5 for European Americans criteria was fulfilled for all 15 SNPs; the MAF between 0.21 and 0.5 for African Americans criteria was fulfilled all 15 SNPs).

*Table 6. SNPs frequency according the origin of populations*

| | Frequency EVS[1] | | Frequency ExAC[2] | | | | | |
|---|---|---|---|---|---|---|---|---|
| SNP | EA[3] | AA[4] | African | East Asian | European (Finnish) | European (Non-Finnish) | Latino | South Asian |
| rs11702450 | 0.3928 | 0.2161 | 0.2095 | 0.1379 | 0.3147 | 0.3973 | 0.2305 | 0.3654 |
| rs843345 | 0.4984 | 0.4251 | 0.5737 | 0.6037 | 0.5042 | 0.5125 | 0.4410 | 0.5732 |
| rs1058018 | 0.4245 | 0.4555 | 0.5498 | 0.7471 | 0.5973 | 0.5700 | 0.5210 | 0.7023 |
| rs8017 | 0.4680 | 0.2882 | 0.2769 | 0.3340 | 0.4339 | 0.5206 | 0.5298 | 0.5167 |
| rs3738494 | 0.4031 | 0.3515 | 0.6509 | 0.6563 | 0.5940 | 0.5991 | 0.5815 | 0.7007 |
| rs1065483 | 0.4089 | 0.4078 | 0.6120 | 0.9730 | 0.5907 | 0.4112 | 0.5131 | 0.7385 |
| rs2839181 | 0.4612 | 0.4988 | 0.4999 | 0.2726 | 0.4881 | 0.4569 | 0.5692 | 0.3960 |
| rs11059924 | 0.4691 | 0.4230 | 0.4324 | 0.5801 | 0.3892 | 0.4708 | 0.3167 | 0.4881 |
| rs2075144 | 0.4577 | 0.4710 | 0.5252 | 0.5646 | 0.4606 | 0.5352 | 0.3808 | 0.7306 |
| rs6795772 | 0.4413 | 0.4055 | 0.3990 | 0.9510 | 0.5975 | 0.5491 | 0.7971 | 0.8323 |
| rs456261 | 0.4705 | 0.4572 | 0.5348 | 0.3280 | 0.5365 | 0.5350 | 0.4968 | 0.6046 |
| rs1131620 | 0.4216 | 0.4366 | 0.5742 | 0.4014 | 0.5232 | 0.4285 | 0.2949 | 0.5575 |
| rs2231926 | 0.4860 | 0.3692 | 0.6209 | 0.4351 | 0.4826 | 0.5102 | 0.3558 | 0.6245 |
| rs352169 | 0.4523 | 0.3340 | 0.3334 | 0.3939 | 0.5022 | 0.5484 | 0.5251 | 0.3940 |
| rs3739160 | 0.4357 | 0.4809 | 0.5500 | 0.6640 | 0.5830 | 0.4738 | 0.5164 | 0.4609 |

[1] Exome Variant Server, http://evs.gs.washington.edu/EVS/
[2] Exome Aggregation Consortium (ExAC), http://exac.broadinstitute.org/
[3] European American
[4] African American

Considering these frequencies, the risk P that at least 2 patients among N patients present with the same SNP profile according to size of series and origin's of patient is shown in Table 7a (according to EVS frequencies) and 7b (according to ExAC frequencies). The risk is less than 5% for series of 96 patients whatever the origin of population (according to the Exome Variant Server and the Exome Aggregation Consortium). The lowest risk is calculated for European population: 0.002174 and 0.002129 for EA EVS frequency and European (Non-Finnish) ExAC frequency, respectively; the highest risk 0.035574 is calculated for East Asian population.

*Table 7. Risk P that at least 2 patients among N patients present the same SNP profile according to size of series and origin's of patient*

a.

| | P | |
|---|---|---|
| Number of patients per series | EA | AA |
| 12 | 0.000032 | 0.000055 |
| 24 | 0.000132 | 0.000231 |
| 48 | 0.000538 | 0.000943 |
| 96 | 0.002174 | 0.003806 |

b.

| | P | | | | | |
|---|---|---|---|---|---|---|
| Number of patients per series | African | East Asian | European (Finnish) | European (Non-Finnish) | Latino | South Asian |
| 12 | 0.000058 | 0.000524 | 0.000036 | 0.000031 | 0.000069 | 0.000092 |
| 24 | 0.000242 | 0.002190 | 0.000149 | 0.000129 | 0.000287 | 0.000383 |
| 48 | 0.000990 | 0.008920 | 0.000609 | 0.000527 | 0.001171 | 0.001565 |
| 96 | 0.003995 | 0.035574 | 0.002459 | 0.002129 | 0.004724 | 0.006313 |

The primers designed for allele-specific multiplex PCR SNP profiling assay are listed in Tables 4 and 5 of the general description above. The sense strand primers comprise only one Locked Nucleic Acid (LNA) base at their 3' end, excepted for primers RABEP1_2457GL2_F_Fam, LTBP4_2359GL2_R_Fam, and PPP4R2_420-1015GL2_R_Fam which are 3' ended by two LNA bases. The sense strand primers specific for allele 2 have 3 additional bases at their 5' end as compared with sense strand primers specific for allele 1. For each SNP, these bases were chosen in order that they do not induce the formation of dimer with neither both the sense strand primers nor the opposite strand primer.

The theoretical size of amplicon, melting temperature of primers, number of bases per primer, number of specific bases per primer are shown in Table 8. The average melting temperature of primers is 65.38°C [62.2°C - 70.9 °C].

*Table 8. Features of primers and amplicons*

| SNP | Name of the primer | Amplicon size (bp)[1] | Tm (°C)[2] | Number of bases per primer | Number of specific bases per primer[3] |
|---|---|---|---|---|---|
| rs11702450 | MCM3AP_1323CL_F_Fam | 103 | 66.0 | 22 | 22 |
| | MCM3AP_1323TL_F_Fam | 106 | 65.4 | 25 | 22 |
| | MCM3AP_ex2_q7_R | - | 65.0 | 29 | 22 |
| rs843345 | ABCF3_837-34TL_R_Fam | 111 | 64.9 | 23 | 23 |
| | ABCF3_837-34CL_R_Fam | 114 | 67.7 | 26 | 24 (+G) |
| | ABCF3_q7_F | - | 64.2 | 30 | 24 (+T) |
| rs1058018 | UBE2Z_846CL_R_Fam | 119 | 66.1 | 20 | 20 |
| | UBE27_846TL_R_Fam | 122 | 64.4 | 23 | 20 |
| | UBE2Z_q7_F | - | 64.8 | 29 | 22 |
| rs8017 | TCEB2_386GL_R_Fam | 127 | 65.3 | 21 | 21 |

(continued)

| SNP | Name of the primer | Amplicon size (bp)[1] | Tm (°C)[2] | Number of bases per primer | Number of specific bases per primer[3] |
|---|---|---|---|---|---|
| | TCEB2_386AL_R_Fam | 130 | 62.5 | 24 | 21 |
| | TCEB2_q7_F | - | 64.8 | 29 | 22 |
| rs3738494 | PPIH_132-40CL_F_Fam | 135 | 65.4 | 20 | 20 |
| | PPIH_132-40TL_F_Fam | 138 | 65.5 | 23 | 21 (+A) |
| | PPIH_q7_R | - | 66.3 | 26 | 19 |
| rs1065483 | RABEP1_2457GL2_F_Fam | 143 | 65.4 | 24 | 24 |
| | RABEP1_2457AL_F_Fam | 146 | 64.6 | 27 | 25 (+A) |
| | RABEP1_q7_R | - | 67.0 | 29 | 22 |
| rs2839181 | MCM3AP_2931TL_R_Fam | 151 | 64.7 | 21 | 21 |
| | MCM3AP_2931CL_R_Fam | 154 | 67.6 | 24 | 21 |
| | MCM3AP_q7_F | - | 64.1 | 29 | 22 |
| rs11059924 | SLC15A4_1245GL_F_Fam | 159 | 65.8 | 23 | 23 |
| | SLC15A4_1245AL_F_Fam | 162 | 64.2 | 26 | 23 |
| | SLC15A4_q7_R | - | 66.0 | 34 | 28 (+T) |
| rs2075144 | PPP5C_363+40GL_R_Fam | 167 | 65.2 | 20 | 20 |
| | PPP5C_363+40AL_R_Fam | 170 | 62.3 | 23 | 20 |
| | PPP5C_q7_F | - | 64.9 | 33 | 26 |
| rs6795772 | USP4_230-20GL_F_Fam | 175 | 65.4 | 26 | 26 |
| | USP4_230-20AL_F_Fam | 178 | 62.2 | 29 | 26 |
| | USP4_q7_R | - | 67.3 | 28 | 21 |
| rs456261 | PFDN6_261-50GL_R_Fam | 183 | 66.0 | 30 | 30 |
| | PFDN6_261-50AL_R_Fam | 186 | 67.0 | 33 | 32 (+GA) |
| | PFDN6_q7_F | - | 66.1 | 31 | 24 |
| rs1131620 | LTBP4_2359AL_R_Fam | 191 | 68.3 | 19 | 19 |
| | LTBP4_2359GL2_R_Fam | 194 | 70.9 | 22 | 19 |
| | LTBP4_q7_F | - | 66.4 | 27 | 20 |
| rs2231926 | PPP4R2_420-1015AL_R_ Fam | 207 | 65.6 | 23 | 23 |
| | PPP4R2_420-1015GL2_R_ Fam | 210 | 66.1 | 26 | 23 |
| | PPP4R2_q7_F | | 68.7 | 32 | 27 (+TT) |
| rs352169 | ALAS1_427+12GL_F_Fam | 215 | 63.2 | 24 | 24 |
| | ALAS1_427+12AL_F_Fam | 218 | 63.5 | 27 | 24 |
| | ALAS1_q7_R | | 65.4 | 28 | 21 |
| rs3739160 | MRPS9_135+31CL_F_Fam | 247 | 63.2 | 21 | 21 |
| | MRPS9_135+31TL_F_Fam | 250 | 62.6 | 24 | 21 |
| | MRPS9_q7_R | | 64.3 | 30 | 23 |

[1] Theoretical size of the PCR product (or amplicon) expressed in base pairs (bp)
[2] Melting temperature of the specific part of the primer
[3] Sense strand primer for allele 1 and opposite strand primer have been designed in a first step. In a second step, sense strand primer for allele 2 was designed by addition of three bases at the 5'end of the sense strand primer for allele 1. In a third step, GTTTCTT sequence was added at the 5' end of the opposite stand primer. These additional bases sometimes resulted in supplementary specific base(s). These supplementary specific base(s) are indicated in brackets.

Optimized PCR conditions are as follows. Primer mix contains the 45 primers at concentration listed in Table 9. The composition of mix PCR is detailed in Table 10. Mix PCR was subjected to PCR amplification: after initial denaturation step (95°C, 15 min), 30 cycles (denaturation 94°C, 30 s; annealing 65°C, 3 min; elongation 72°C, 90 s) were performed, followed by final elongation step (72°C, 10 min). PCR products were stored on PCR thermal cycler at 10°C until storage at 4°C. One μl of PCR products diluted 50 to 200 fold in water for injection was mixed with 15 μl of ROX-Formamide mix prepared as follows: 0.1 μl of GeneScan™ 400HD ROX™ Size Standard (Applied Biosystem®, by Life Technologies™) was added to 15 μl of Hi-Di formamide genetic analysis grade (Life Technologies™). The resulting mix -diluted PCR product-ROX-formamide- was loaded on a 3730 DNA analyzer. Samples were run with the following parameters: oven temperature 66°C, pre-run voltage 15 kV, injection voltage 2 kV, injection time 3 s, Dye set Any4Dye-HDR or Any4Dye.

*Table 9. Primer concentration in the primer mix.*

| Reagent | Primer Concentration in the primer mix (μM) |
|---|---|
| MCM3AP_1323CL_F_Fam | 1 |
| MCM3AP_1323TL_F_Fam | 1 |
| MCM3AP_ex2_q7_R | 1 |
| ABCF3_837-34TL_R_Fam | 1 |
| ABCF3_837-34CL_R_Fam | 0.5 |
| ABCF3_q7_F | 1 |
| UBE2Z_846CL_R_Fam | 1 |
| UBE27_846TL_R_Fam | 0.5 |
| UBE2Z_q7_F | 1 |
| TCEB2_386GL_R_Fam | 1 |
| TCEB2_386AL_R_Fam | 1 |
| TCEB2_q7_F | 1 |
| PPIH_132-40CL_F_Fam | 1 |
| PPIH_132-40TL_F_Fam | 1 |
| PPIH_q7_R | 1 |
| RABEP1_2457GL2_F_Fam | 1.5 |
| RABEP1_2457AL_F_Fam | 1 |
| RABEP1_q7_R | 1 |
| MCM3AP_2931TL_R_Fam | 1 |
| MCM3AP_2931CL_R_Fam | 0.3 |
| MCM3AP_q7_F | 1 |
| SLC15A4_1245GL_F_Fam | 1 |
| SLC15A4_1245AL_F_Fam | 1 |
| SLC15A4_q7_R | 1 |
| PPP5C_363+40GL_R_Fam | 2 |
| PPP5C_363+40AL_R_Fam | 1 |
| PPP5C_q7_F | 1 |
| USP4_230-20GL_F_Fam | 1 |
| USP4_230-20AL_F_Fam | 0.75 |
| USP4_q7_R | 1 |
| PFDN6_261-50GL_R_Fam | 1 |
| PFDN6_261-50AL_R_Fam | 1 |
| PFDN6_q7_F | 1 |
| LTBP4_2359AL_R_Fam | 1 |
| LTBP4_2359GL2_R_Fam | 2 |
| LTBP4_q7_F | 1 |
| PPP4R2_420-1015AL_R_Fam | 0.75 |
| PPP4R2_420-1015GL2_R_Fam | 1 |
| PPP4R2_q7_F | 1 |
| ALAS1_427+12GL_F_Fam | 2 |
| ALAS1_427+12AL_F_Fam | 2 |
| ALAS1_q7_R | 2 |
| MRPS9_135+31CL_F_Fam | 1 |

(continued)

| Reagent | Primer Concentration in the primer mix (μM) |
|---|---|
| MRPS9_135+31TL_F_Fam | 1 |
| MRPS9_q7_R | 1 |
| Buffer ATE* | to the above concentration |
| * reagent of QIAsymphony DSP DNA Midi Kit (96) (Cat No./ID: 937255) | |

*Table 10. Composition of mix PCR*

| Reagent | Volume for 1 reaction (μl) |
|---|---|
| RNase-Free Water (Qiagen) [1] | 14 |
| 5x Q-Solution (Qiagen) [1] | 5 |
| 2x QIAGEN Multiplex PCR Master Mix (Qiagen) [1] | 25 |
| Primer Mix[2] | 5 |
| DNA (minimum 50ng/μl) [3] | 1 |
| Final volume | 50 |

[1] QIAGEN Multiplex PCR Kit (100 reactions: Cat No./ID: 206143; 1000 reactions: Cat No./ID: 206145)
[2] see Table 9
[3] 2μl or 3μl DNA if DNA concentration is <25ng/μl

Raw data were analyzed by GeneMapper™ Software 5 software. A specific analysis method, based on the "OLA Analysis" analysis type, and a binset have been created allowing the labeling of the peaks. The results can be viewed in two ways: electrophoregram with labeling of each peak or table showing which alleles were identified for each patient. Figure 2 shows illustrative electrophoregrams of the SNP profiling assay described above for three patients. The genotype can be easily determined for each SNP by the presence of: only one peak corresponding to allele 1 (genotype 1/1), or only one peak corresponding to allele 2 (genotype 2/2), or the presence of two peaks, corresponding to allele 1 and 2 (genotype 1/2). No "plus-A artifact" is observed. For the three electrophoregrams shown in Figure 2, the corresponding SNP profile is detailed in Table 11a and 11b.

The results presented in table format with GeneMapper™ Software 5 software were exported in a .txt format.

*Table 11. Interpretation of electrophoregrams in Figure 2*

a. Genotype of SNP according to the electrophoregrams shown in Figure 2

| SNP | Patient 1 | Patient 2 | Patient 3 |
|---|---|---|---|
| 01 | 1/2 | 1/1 | 2/2 |
| 02 | 1/2 | 1/2 | 2/2 |
| 03 | 1/2 | 2/2 | 2/2 |
| 04 | 1/1 | 1/2 | 1/2 |
| 05 | 1/2 | 2/2 | 1/2 |
| 06 | 1/2 | 2/2 | 2/2 |
| 07 | 1/2 | 2/2 | 1/1 |
| 08 | 1/2 | 1/1 | 1/1 |
| 09 | 1/2 | 1/2 | 1/1 |
| 10 | 1/1 | 2/2 | 1/2 |
| 11 | 1/2 | 2/2 | 1/2 |
| 12 | 1/2 | 2/2 | 1/2 |
| 13 | 1/1 | 1/1 | 1/1 |
| 14 | 1/2 | 1/2 | 2/2 |
| 15 | 1/2 | 1/2 | 1/1 |

(continued)

| b. Same results expressed for each SNP with reference base and/or alternate base according to the gene sense of transcription (see Table 2.) | | | |
|---|---|---|---|
| SNP | Patient 1 | Patient 2 | Patient 3 |
| rs11702450 | C/T | C/C | T/T |
| rs843345 | T/C | T/C | C/C |
| rs1058018 | C/T | T/T | T/T |
| rs8017 | G/G | G/A | G/A |
| rs3738494 | C/T | T/T | C/T |
| rs1065483 | G/A | A/A | A/A |
| rs2839181 | T/C | C/C | T/T |
| rs11059924 | G/A | G/G | G/G |
| rs2075144 | G/A | G/A | G/G |
| rs6795772 | G/G | A/A | G/A |
| rs456261 | G/A | A/A | G/A |
| rs1131620 | A/G | G/G | A/G |
| rs2231926 | A/A | A/A | A/A |
| rs352169 | G/A | G/A | A/A |
| rs3739160 | C/T | C/T | C/C |
| 1/1: homozygous for the reference allele; 2/2: homozygous for the alternate allele; 1/2 heterozygous for reference and alternate allele | | | |

One of the expected features of the SNP profiling assay was to have an assay that can be used routinely. This can be achieved only if the assay is simple to run, in the present case if the number of reagents to mix in the PCR reactions is not too high. As 45 primers are needed to determine the SNP profile for the 15 SNPs, it was necessary to simplify the PCR preparation by using pre-prepared mixes containing at least the 45 primers. However, such pre-prepared mixes have to demonstrate a good stability in time. Hence, test of stability of two mixes have been performed: the first mix contains only the primers (primer mix) and the second one contains all the PCR reagents and primers excepted DNA (mix-PCR). The two mixes have been prepared, aliquoted in suitable volume, and frozen for twelve months. Both mixes have been tested over a period of 12 months (one test per month for each mix) with remarkable stability of the results, as demonstrated by the overlay of monthly electrophoregrams for each sample tested (data not shown). This perfect stability observed at twelve months is encouraging and demonstrates that mixes can be prepared in batch, aliquoted, frozen, and used at least 12 months after the production date, which is suited to a routinely application.

The robustness of the SNP profiling assay is satisfying considering the four variables tested. Indeed, the results obtained using different PCR thermal cyclers are similar to each other. The quality of results from DNA extracted with two different procedures is satisfying for both. The use of an amount of 10 to 400 ng DNA per test showed good quality of results, whatever the initial amount of DNA. This latter point is of particular importance as one of the requirement for the SNP profiling assay was the use of primary DNA samples, whatever their DNA concentration. Finally, results obtained with 1 μl of primary DNA sample mixed with 9μl, 14μl, 24μl or 49μl of mix-PCR are similar if primary DNA sample concentration ranged from 10 to 400 ng/μl. Therefore, we now perform the SNP profiling assay routinely with 9μl of mix-PCR and 1μl of primary DNA sample if primary DNA sample concentration is bellow or equal to 400 ng/μl (which is the very most frequent situation); if DNA concentration is higher, we recommend to mix 24μl of mix-PCR with 1μl of primary DNA sample to perform the test.

**NGS assay**

In order to perform NGS assay including the selected SNP, the manufacturer of our NGS capture custom kit (Roche NimbleGen Inc.) was requested to add the probes corresponding to the SNPs in a new version of our custom kit according to the coordinates of the regions of interest listed in Table 12. We defined the regions of interest as 100 base pairs by each side of the SNP coordinate.

*Table 12. Coordinates of regions of interest for probe design*

| SNP | Chromosome | 5' end of the region of interest[1] | 3' end of the region of interest[1] | Size of the region of interest (pb) | SEQ ID NO |
|---|---|---|---|---|---|
| rs11702450 | chr21 | 47703549 | 47703749 | 201 | SEQ ID NO: 46 |
| rs843345 | chr3 | 183906415 | 183906615 | 201 | SEQ ID NO: 47 |
| rs1058018 | chr17 | 47000151 | 47000351 | 201 | SEQ ID NO: 48 |
| rs8017 | chr16 | 2821473 | 2821673 | 201 | SEQ ID NO: 49 |
| rs3738494 | chr1 | 43124759 | 43124959 | 201 | SEQ ID NO: 50 |
| rs1065483 | chr17 | 5284670 | 5284870 | 201 | SEQ ID NO: 51 |
| rs2839181 | chr21 | 47685839 | 47686039 | 201 | SEQ ID NO: 52 |
| rs11059924 | chr12 | 129293246 | 129293446 | 201 | SEQ ID NO: 53 |
| rs2075144 | chr19 | 46857186 | 46857386 | 201 | SEQ ID NO: 54 |
| rs6795772 | chr3 | 49365169 | 49365369 | 201 | SEQ ID NO: 55 |
| rs456261 | chr6 | 33258343 | 33258543 | 201 | SEQ ID NO: 56 |
| rs1131620 | chr19 | 41117769 | 41117969 | 201 | SEQ ID NO: 57 |
| rs2231926 | chr3 | 73111709 | 73111909 | 201 | SEQ ID NO: 58 |
| rs352169 | chr3 | 52236662 | 52236862 | 201 | SEQ ID NO: 59 |
| rs3739160 | chr2 | 105654616 | 105654816 | 201 | SEQ ID NO: 60 |
| [1] Human assembly GRCh37/hg19 coordinates | | | | | |

The new custom kit was used as usually to test series of 24 patients. After carrying out the NGS sequencing, the raw data were transferred to the Genodiag company for bioinformatics analysis. For the 15 SNPs, the company Genodiag provided a summary of the results in tabular form, allowing an easy reading of the genotype of the SNP combination (Table 13). The number of reads for each SNP was more than 30X.

*Table 13. Results of the genotype of the SNP combination obtained by NGS assay*

| SNP | Patient 01-M | Patient 02-F | Patient 03-M | Patient 04-M | Patient 05-F | Patient 06-F | Patient 07-M | Patient 08-M | Patient 09-M | Patient 10-F | Patient 11-M | Patient 12-M | Patient 13-M | Patient 14-M | Patient 15-F | Patient 16-F | Patient 17-F | Patient 18-F | Patient 19-M | Patient 20-M | Patient 21-M | Patient 22-M | Patient 23-M | Patient 24-F |
|---|---|---|---|---|---|---|---|---|---|---|---|---|---|---|---|---|---|---|---|---|---|---|---|---|
| 1 | C/C | C/T | C/T | C/T | C/T | C/C | C/T | C/T | C/C | C/T | C/T | C/T | C/T | C/T | C/T | C/C | C/C | C/T | C/T | C/C | C/C | C/T | C/T | C/C |
| 2 | T/C | T/C | T/C | T/T | C/C | C/C | C/C | C/C | T/T | T/C | T/T | T/T | C/C | C/C | T/C | T/C | C/C | C/C | C/C | T/C | C/C | T/C | C/C | C/C |
| 3 | T/T | C/C | C/T | C/C | T/T | T/T | C/T | C/T | C/T | T/T | T/T | C/T | T/T | C/C | T/T | C/C | T/T | C/T | T/T | C/C | C/C | C/T | C/C | C/C |
| 4 | G/A | G/A | G/G | G/G | G/A | G/A | G/A | G/A | G/A | A/A | A/A | G/A | G/G | G/A | G/A | A/A | G/G | G/G | G/A | G/A | G/G | G/G | A/A | G/G |
| 5 | C/T | C/T | C/C | C/C | C/T | C/T | T/T | C/C | C/C | T/T | C/T | C/T | C/T | C/T | T/T | T/T | C/T | C/C | T/T | T/T | T/T | C/C | T/T | C/C |
| 6 | G/G | G/G | A/A | G/A | G/G | G/A | G/A | G/A | G/G | G/G | G/A | G/A | G/A | G/G | G/A | G/A | G/G | G/G | A/A | G/A | G/G | A/A | G/A | G/A |
| 7 | T/C | T/C | T/T | T/C | T/C | T/C | T/T | T/C | T/C | T/C | T/T | T/T | T/C | T/C | T/C | T/T | T/C | T/T | T/C | T/C | C/C | T/T | T/C | C/C |
| 8 | G/A | G/A | A/A | G/G | G/G | A/A | G/A | G/G | G/A | G/A | A/A | G/G | G/A | A/A | G/G | G/A | G/G | G/G | A/A | A/A | G/A | A/A | G/G | G/A |
| 9 | G/A | G/G | G/A | G/G | G/A | G/A | A/A | A/A | A/A | A/A | A/A | G/G | A/A | G/A | G/A | A/A | G/G | A/A | G/A | G/A | G/A | G/A | G/A | A/A |
| 10 | G/G | A/A | A/A | G/G | G/G | A/A | G/A | G/A | G/A | A/A | G/G | G/A | G/A | A/A | G/A | A/A | G/A | G/G | G/A | G/G | G/G | A/A | A/A | G/A |
| 11 | G/A | G/A | G/G | A/A | A/A | G/G | A/A | A/A | A/A | G/A | G/G | A/A | G/A | A/A | G/A | G/A | G/G | G/A | A/A | G/A | G/G | G/G | G/G | A/A |
| 12 | G/G | A/G | A/A | A/A | A/A | A/G | A/A | A/A | A/A | A/A | A/G | A/A | A/A | A/G | A/G | A/A | A/A | A/G | A/G | G/G | A/A | A/A | A/G | G/G |
| 13 | A/G | A/G | G/G | G/G | G/G | G/G | A/G | A/G | A/G | A/G | A/G | A/A | A/G | A/G | G/G | G/G | A/G | G/G | A/G | A/G | A/A | G/G | A/A | A/G |
| 14 | G/A | G/G | G/G | G/A | A/A | G/A | G/A | G/A | A/A | A/A | G/A | G/A | G/A | A/A | G/G | G/A | G/G | G/A | G/A | G/G | G/A | G/G | G/A | A/A |
| 15 | C/T | C/T | T/T | C/T | C/T | C/T | C/C | C/T | C/C | C/C | T/T | C/C | C/C | C/T | C/C | C/T | C/T | C/T | C/T | T/T | C/T | T/T | C/C | C/C |
| SRY | M | F | M | M | F | F | M | M | M | F | M | M | M | M | F | F | F | F | M | M | M | M | M | M |

SNP are numbered according to Table 2. SRY is a gene located on Y chromosome; probe corresponding to this gene was previously included in the custom kit in order to test for the sex of patient, which participates to the sample tracking. In order to illustrate the different possible discrepancies, SNP results for Patients 05 and 15 have been switched; columns for Patients 19 and 21 have been switched; sex of Patient 24 have been modified in the label of the corresponding column; results for Patient 22 have been replaced by results of Patient 03.

**Comparison of SNP profile obtained by allele-specific Multiplex PCR SNP profiling assay and NGS assay; Interpretation**

Validation of NGS genotyping results is provided by the comparison of SNP profile obtained by allele-specific Multiplex PCR SNP profiling assay and NGS assay. The NGS genotyping results, provided that they passed quality control and threshold filters, and that bioinformatics pipeline provides accurate nomenclature of variants, do not need to be confirmed by another technique if the results of the SNP profiling assay is strictly identical to the corresponding NGS genotyping results, and if none of the patients from the series present an identical SNP profile. Results of SNP profiling assay not strictly identical to NGS genotyping results will reveal sample mix-up: in this case, NGS genotyping results cannot be validated. If two patients have the same SNP profile, either they are really different people -which would be revealed by NGS genotyping results showing many differences- and Sanger sequencing would have to be subsequently performed to validate their own NGS genotyping results or, the same DNA samples have been tested with two different identifiers, a situation that will be revealed by identical NGS genotyping results for the two identifiers and that will reveal sample mix-up. In this latter case, NGS genotyping results cannot be validated.

In order to facilitate the comparison of results of SNP profile obtained by NGS assay with those of allele-specific PCR SNP profiling assay, an Excel file has been created. This file consists of four visible worksheets. The first worksheet is used to paste the results of NGS such as those presented in Table 13. The second worksheet allows to paste the .txt file exported from Genemapper (non visible additional worksheets allow to transform Genemapper results in a suitable format for comparison with NGS genotyping results). The third worksheet (see example in Table 14.) allows to compare: 1) the order of samples in the work list of the NGS assay results with that of the allele-specific PCR SNP profiling assay (if the patient identifier is identical to the position considered for both techniques, it appears in clear text; in the event of discrepancy, words "erreur ordre" replaces the patient's identifier); 2) the genotype obtained by NGS assay and that obtained by allele-specific PCR SNP profiling assay for each SNP (if the genotype is identical, it appears in clear text; in the case of discordance, word *"Pb"* replaces the genotype); 3) the sex of the patient determined by NGS assay (SRY line in Table 13.) with the sex of the patient indicated on the patient ID of the work list (if the sex is identical, the letter "*F*" or "*M*" appear in clear text for female and male, respectively; in case of discrepancy, the word *"Pb"* replaces the gender). Table 14 illustrates the different possible discrepancies. As expected after the intentional modifications of NGS genotyping results depicted in legend to Table 13., the SNP profiles obtained by NGS assay and allele-specific PCR SNP profiling assay for patient 05 and patient 15 are not identical (8 discrepancies for each); the patient identifier is different in the NGS assay work list as compared to SNP profiling assay work list at the position corresponding to patients 19 and 21; the SNP profiles (NGS vs SNP profiling assay) are not identical for patient 19 as well as for patient 21 (10 discrepancies for each); sex of Patient 24 is F in the work list whereas the sex determined by NGS assay is M.

*Table 14. Table simulating the comparison of the results of NGS and the allele-specific PCR, illustrating the different possible discrepancies*

| Patient → / SNP↓ | Patient 01-M | Patient 02-F | Patient 03-M | Patient 04-M | Patient 05-F | Patient 06-F | Patient 07-M | Patient 08-M | Patient 09-M | Patient 10-F | Patient 11-M | Patient 12-M | Patient 13-M | Patient 14-M | Patient 15-F | Patient 16-F | Patient 17-F | Patient 18-F | erreur ordre | Patient 20-M | erreur ordre | Patient 22-M | Patient 23-M | Patient 24-F |
|---|---|---|---|---|---|---|---|---|---|---|---|---|---|---|---|---|---|---|---|---|---|---|---|---|
| 01 | C/C | C/T | C/T | C/T | C/T | C/C | C/T | C/T | C/C | C/T | C/T | C/T | C/T | C/T | C/T | C/C | C/C | C/T | Pb | C/C | Pb | C/T | C/T | C/C |
| 02 | T/C | T/C | T/C | T/T | Pb | C/C | C/C | C/C | T/T | T/C | T/T | T/T | C/C | C/C | Pb | T/C | C/C | C/C | C/C | T/C | C/C | T/C | C/C | C/C |
| 03 | T/T | C/C | C/T | C/C | T/T | T/T | C/T | C/T | C/T | T/T | T/T | C/T | T/T | C/C | T/T | C/C | T/T | C/T | Pb | C/C | Pb | C/T | C/C | C/C |
| 04 | G/A | G/A | G/G | G/G | G/A | G/A | G/A | G/A | G/A | A/A | A/A | G/A | G/G | G/A | G/A | A/A | G/G | G/G | Pb | G/A | Pb | G/G | A/A | G/G |
| 05 | C/T | C/T | C/C | C/C | Pb | C/T | T/T | C/C | C/C | T/T | C/T | C/T | C/T | C/T | Pb | T/T | C/T | C/C | T/T | T/T | T/T | C/C | T/T | C/C |
| 06 | G/G | G/G | A/A | G/A | Pb | G/A | G/A | G/A | G/G | G/G | G/A | G/A | G/A | G/G | Pb | G/A | G/G | G/G | Pb | G/A | Pb | A/A | G/A | G/A |
| 07 | T/C | T/C | T/T | T/C | T/C | T/C | T/T | T/C | T/C | T/C | T/T | T/T | T/C | T/C | T/C | T/T | T/C | T/T | Pb | T/C | Pb | T/T | T/C | C/C |
| 08 | G/A | G/A | A/A | G/G | G/G | A/A | G/A | G/G | G/A | G/A | A/A | G/G | G/A | A/A | G/G | G/A | G/G | G/G | Pb | A/A | Pb | A/A | G/G | G/A |
| 09 | G/A | G/G | G/A | G/G | G/A | G/A | A/A | A/A | A/A | A/A | A/A | G/G | A/A | G/A | G/A | A/A | G/G | A/A | G/A | G/A | G/A | G/A | G/A | A/A |
| 10 | G/G | A/A | A/A | G/G | Pb | A/A | G/A | G/A | G/A | A/A | G/G | G/A | G/A | A/A | Pb | A/A | G/A | G/G | Pb | G/G | Pb | A/A | A/A | G/A |
| 11 | G/A | G/A | G/G | A/A | Pb | G/G | A/A | A/A | A/A | G/A | G/G | A/A | G/A | A/A | Pb | G/A | G/G | G/A | Pb | G/A | Pb | G/G | G/G | A/A |
| 12 | G/G | A/G | A/A | A/A | Pb | A/G | A/A | A/A | A/A | A/A | A/G | A/A | A/A | A/G | Pb | A/A | A/A | A/G | Pb | G/G | Pb | A/A | A/G | G/G |
| 13 | A/G | A/G | G/G | G/G | G/G | G/G | A/G | A/G | A/G | A/G | A/G | A/A | A/G | A/G | G/G | G/G | A/G | G/G | Pb | A/G | Pb | G/G | A/A | A/G |
| 14 | G/A | G/G | G/G | G/A | Pb | G/A | G/A | G/A | A/A | A/A | G/A | G/A | G/A | A/A | Pb | G/A | G/G | G/A | G/A | G/G | G/A | G/G | G/A | A/A |
| 15 | C/T | C/T | T/T | C/T | Pb | C/T | C/C | C/T | C/C | C/C | T/T | C/C | C/C | C/T | Pb | C/T | C/T | C/T | C/T | T/T | C/T | T/T | C/C | C/C |
| SRY | M | F | M | M | F | F | M | M | M | F | M | M | M | M | F | F | F | F | M | M | M | M | M | Pb |

The fourth worksheet is an Excel PivotTable based on the previous worksheet. It allows to determine how many patients presented with the same SNP profile, i.e. the same combination for the 15 SNPs (Table 15.): the "Total Général" column indicates the number of patients with the same SNP profile (resulted from the genotype shown for each SNP in columns 1 to 15). Here, Patient 03-M and Patient 22-M have the same SNP profile, which was difficult to see in Table 14 (again, this result was expected after the intentional modification of NGS genotyping results depicted in legend to Table 13.).

*Table 15. PivotTable to determine how many patients presented with the same SNP profile*

| 01 | 02 | 03 | 04 | 05 | 06 | 07 | 08 | 09 | 10 | 11 | 12 | 13 | 14 | 15 | erreur ordre | Patient 01-M | Patient 02-F | Patient 03-M | Patient 04-M | Patient 05-F | Patient 06-F | Patient 07-M | Patient 08-M | Patient 09-M | Patient 10-F | Patient 11-M | Patient 12-M | Patient 13-M | Patient 14-M | Patient 15-F | Patient 16-F | Patient 17-F | Patient 18-F | Patient 20-M | Patient 22-M | Patient 23-M | Patient 24-F | Total général |
|---|---|---|---|---|---|---|---|---|---|---|---|---|---|---|---|---|---|---|---|---|---|---|---|---|---|---|---|---|---|---|---|---|---|---|---|---|---|---|
| C/C | C/C | C/C | G/G | C/C | G/A | C/C | G/A | A/A | G/A | A/A | G/G | A/G | A/A | C/C | | | | | | | | | | | | | | | | | | | | | | | 1 | 1 |
| C/C | C/C | T/T | G/A | C/T | G/A | T/C | A/A | G/A | A/A | G/G | A/G | G/G | G/A | C/T | | | | | | | 1 | | | | | | | | | | | | | | | | | 1 |
| C/C | C/C | T/T | G/G | C/T | G/G | T/C | G/G | G/G | G/A | G/G | A/A | A/G | G/G | C/T | | | | | | | | | | | | | | | | | | 1 | | | | | | 1 |
| C/C | T/C | C/C | A/A | T/T | G/A | T/T | G/A | A/A | A/A | G/A | A/A | G/G | G/A | C/T | | | | | | | | | | | | | | | | | 1 | | | | | | | 1 |
| C/C | T/C | C/C | G/A | T/T | G/A | T/C | A/A | G/A | G/G | G/A | G/G | A/G | G/G | T/T | | | | | | | | | | | | | | | | | | | | 1 | | | | 1 |
| C/C | T/C | T/T | G/A | C/T | G/G | T/C | G/A | G/A | G/G | G/A | G/G | A/G | G/A | C/T | | 1 | | | | | | | | | | | | | | | | | | | | | | 1 |
| C/C | T/T | C/T | G/A | C/C | G/G | T/C | G/A | A/A | G/A | A/A | A/A | A/G | A/A | C/C | | | | | | | | | | 1 | | | | | | | | | | | | | | 1 |
| C/T | C/C | C/C | A/A | T/T | G/A | T/C | G/G | G/A | A/A | G/G | A/G | A/A | G/A | C/C | | | | | | | | | | | | | | | | | | | | | | 1 | | 1 |
| C/T | C/C | C/C | G/A | C/T | G/G | T/C | A/A | G/A | A/A | A/A | A/G | A/G | A/A | C/T | | | | | | | | | | | | | | | 1 | | | | | | | | | 1 |
| C/T | C/C | C/T | G/A | C/C | G/A | T/C | G/G | A/A | G/A | A/A | A/A | A/G | G/A | C/T | | | | | | | | | 1 | | | | | | | | | | | | | | | 1 |
| C/T | C/C | C/T | G/A | T/T | G/A | T/T | G/A | A/A | G/A | A/A | A/A | A/G | G/A | C/C | | | | | | | | 1 | | | | | | | | | | | | | | | | 1 |
| C/T | C/C | C/T | G/G | C/C | G/G | T/T | G/G | A/A | G/G | G/A | A/G | G/G | G/A | C/T | | | | | | | | | | | | | | | | | | | 1 | | | | | 1 |
| C/T | C/C | T/T | G/G | C/T | G/A | T/C | G/A | A/A | G/A | G/A | A/A | A/G | G/A | C/C | | | | | | | | | | | | | | 1 | | | | | | | | | | 1 |
| C/T | Pb | T/T | G/A | Pb | Pb | T/C | G/G | G/A | Pb | Pb | Pb | G/G | Pb | Pb | | | | | | 1 | | | | | | | | | | 1 | | | | | | | | 2 |
| C/T | T/C | C/C | G/A | C/T | G/G | T/C | G/A | G/G | A/A | G/A | A/G | A/G | G/G | C/T | | | 1 | | | | | | | | | | | | | | | | | | | | | 1 |
| C/T | T/C | C/T | G/G | C/C | A/A | T/T | A/A | G/A | A/A | G/G | A/A | G/G | G/G | T/T | | | | 1 | | | | | | | | | | | | | | | | | 1 | | | 2 |
| C/T | T/C | T/T | A/A | T/T | G/G | T/C | G/A | A/A | A/A | G/A | A/A | A/G | A/A | C/C | | | | | | | | | | | 1 | | | | | | | | | | | | | 1 |
| C/T | T/T | C/C | G/G | C/C | G/A | T/C | G/G | G/G | G/G | A/A | A/A | G/G | G/A | C/T | | | | | 1 | | | | | | | | | | | | | | | | | | | 1 |
| C/T | T/T | C/T | G/A | C/T | G/A | T/T | G/G | G/G | G/A | A/A | A/A | A/A | G/A | C/C | | | | | | | | | | | | | 1 | | | | | | | | | | | 1 |
| C/T | T/T | T/T | A/A | C/T | G/A | T/T | A/A | A/A | G/G | G/G | A/G | A/G | G/A | T/T | | | | | | | | | | | | 1 | | | | | | | | | | | | 1 |
| Pb | C/C | Pb | Pb | T/T | Pb | Pb | Pb | G/A | Pb | Pb | Pb | Pb | G/A | C/T | 2 | | | | | | | | | | | | | | | | | | | | | | | 2 |
| Total général | | | | | | | | | | | | | | | 2 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 24 |

***Conclusion***

We designed an allele-specific multiplex PCR SNP profiling assay to validate NGS genotyping results by comparison of SNP profile obtained by both assays. This allele-specific multiplex PCR SNP profiling assay is suited to routine procedure in any genetics laboratory.

Indeed, our allele-specific multiplex PCR SNP profiling assay is rapid: one single PCR reaction followed by capillary electrophoresis allows to determine the SNP profile of a combination of 15 SNPs. Primary DNA samples can be used for the test. Pre-prepared mixes for PCR preparation have demonstrated stability over at least twelve months. SNP profiling assay needs devices (PCR Thermal Cycler and capillary electrophoresis systems) that are usually routinely used in genetics laboratories. The SNP are located in housekeeping gene: therefore, NGS sequencing of the SNP regions of interest cannot lead to unsolicited findings. As the same SNP set can be added in any NGS capture kit or NGS amplicon kit, our allele-specific multiplex PCR SNP profiling assay can be the unique SNP profiling assay used in a laboratory performing NGS assays with different NGS kits. It has a high discrimination power, as the risk for two samples of a series of 96 samples to have the same SNP profile is less than 5%, whatever the origin of patient, reaching 0.2 % in European population (i.e. statistically, NGS genotyping results would have to be confirmed for two patients by Sanger Sequencing only for 2 out of 1000 NGS assays). Interpretation of SNP profiling assay results is simple and rapid. The NGS genotyping results for the 15 SNP are reliable as the coverage reaches more than 30 reads. The SNP profiles obtained by both NGS and allele-specific PCR SNP profiling assays can be easily compared using an Excel file designed for this purpose.

Thus, if the results of the SNP profiling assay is strictly identical to the corresponding NGS genotyping results and if none of the patients from the series present an identical SNP profile, the NGS genotyping results do not need to be confirmed by another technique, which results in a considerable time saving in the laboratory processes.

**Example 2: Implementation of the allele-specific multiplex PCR SNP profiling assay for validation of whole exome sequencing results**

The SNPs of the above described SNP profiling assay are exonic (rs11702450, rs1058018, rs8017, rs1065483, rs2839181, rs11059924, rs1131620) or near the exon-intron +/- 50 bp junction (rs843345, rs3738494, rs2075144, rs6795772, rs456261, rs352169, rs3739160), with the exception of a single SNP (rs2231926) located at a distance from an exon-intron junction (-1015 pb). Therefore, as fourteen out of the 15 SNPs are potentially covered in whole exome sequencing studies (WES), we checked if SNP coverage was sufficient in WES (≥20X). If so, allele-specific multiplex PCR SNP profiling assay could also be used for sample pairing for WES assay.

WES using the SeqCap EZ MedExome Enrichment Kit (Roche, Nimblegen) in a series of 12 patients demonstrates sufficient coverage for all 15 SNPs (Table 16.), including rs2231926 located at -1015 bases from the intron-exon junction. Only one coverage value is less than 20X (15X; patient #2 for rs3739160) (data provided by Dr. Boris Keren, Department of Genetics, Functional Genomics Development Unit, Pitié-Salpêtrière Hospital Group).

These results have to be confirmed in other WES series of patient. Nevertheless, they show that our allele-specific multiplex PCR SNP profiling assay may probably also be used for sample pairing for WES assay.

*Table 16. SNP coverage for a series of 12 patients studied by WES*

| SNP | Cov #1 | Cov #2 | Cov #3 | Cov #4 | Cov #5 | Cov #6 | Cov #7 | Cov #8 | Cov #9 | Cov #10 | Cov #11 | Cov #12 | Mean Cov |
|---|---|---|---|---|---|---|---|---|---|---|---|---|---|
| rs11702450 | 93 | 67 | 85 | 88 | 106 | 104 | 96 | 81 | 93 | 84 | 122 | 81 | 91 |
| rs843345 | 32 | 29 | 43 | 38 | 47 | 43 | 33 | 22 | 46 | 38 | 49 | 33 | 37 |
| rs1058018 | 49 | 52 | 54 | 78 | 56 | 83 | 80 | 66 | 65 | 51 | 57 | 69 | 63 |
| rs8017 | 49 | 45 | 66 | 60 | 70 | 76 | 69 | 54 | 68 | 66 | 61 | 77 | 63 |
| rs3738494 | 42 | 20 | 20 | 26 | 30 | 47 | 20 | 28 | 27 | 34 | 21 | 30 | 28 |
| rs1065483 | 103 | 104 | 89 | 102 | 97 | 115 | 104 | 98 | 94 | 84 | 97 | 118 | 100 |
| rs2839181 | 65 | 55 | 75 | 65 | 72 | 77 | 64 | 71 | 51 | 79 | 105 | 64 | 70 |
| rs11059924 | 132 | 114 | 113 | 132 | 109 | 116 | 125 | 125 | 145 | 111 | 144 | 131 | 124 |
| rs2075144 | 29 | 29 | 38 | 38 | 25 | 48 | 21 | 37 | 25 | 40 | 45 | 32 | 33 |
| rs6795772 | 107 | 72 | 85 | 79 | 100 | 110 | 106 | 97 | 95 | 83 | 98 | 100 | 94 |
| rs456261 | 27 | 25 | 31 | 38 | 27 | 53 | 34 | 25 | 47 | 31 | 41 | 24 | 33 |
| rs1131620 | 40 | 27 | 48 | 50 | 60 | 55 | 42 | 48 | 55 | 59 | 68 | 48 | 50 |
| rs2231926 | 94 | 108 | 107 | 97 | 125 | 127 | 102 | 89 | 117 | 111 | 117 | 119 | 109 |
| rs352169 | 72 | 78 | 63 | 61 | 79 | 84 | 74 | 54 | 55 | 62 | 65 | 78 | 68 |
| rs3739160 | 24 | 15 | 22 | 35 | 26 | 44 | 40 | 23 | 20 | 24 | 36 | 37 | 28 |

**BIBLIOGRAPHIC REFERENCES**

Auer et al., NHLBI GO Exome Sequencing Project. Guidelines for Large-Scale Sequence-Based Complex Trait Association Studies: Lessons Learned from the NHLBI Exome Sequencing Project. Am J Hum Genet. 2016 Oct 6;99(4):791-801

Brownstein MJ, Carpenter JD, Smith JR: Modulation of non-templated nucleotide addition by Taq DNA polymerase: primer modifications that facilitate genotyping. BioTechniques 1996; 20: 1004-1010.

Eisenberg E, Levanon EY. Human housekeeping genes are compact. Trends Genet 2003;19(7):362-5.

Lek M et al. Exome Aggregation Consortium. Analysis of protein-coding genetic variation in 60,706 humans. Nature. 2016 Aug 18;536(7616):285-91

Maniatis N et al. The first linkage disequilibrium (LD) maps: delineation of hot and cold blocks by diplotype analysis. Proc Natl Acad Sci U S A. 2002 Feb 19;99(4):2228-33.

Matthijs G, Souche E, Alders M, et al. Guidelines for diagnostic next-generation sequencing. Eur J Hum Genet 2016;24:2-5.

Pengelly RJ et al. A SNP profiling panel for sample tracking in whole-exome sequencing studies. Genome Med. 5, 89 (2013).

Reich DE et al. Linkage disequilibrium in the human genome. Nature. 2001 May 10;411(6834):199-204

Risch N, Merikangas K. The future of genetic studies of complex human diseases. Science. 1996 Sep 13;273(5281):1516-7.

Voelkerding KV, Dames S, Durtschi JD. Next generation sequencing for clinical diagnostics-principles and application to targeted resequencing for hypertrophic cardiomyopathy: a paper from the 2009 William Beaumont Hospital Symposium on Molecular Pathology. J Mol Diagn. 2010;12(5):539-551.

Zhu J1, He F, Song S, et al. How many human genes can be defined as housekeeping with current expression data? BMC Genomics 2008;9:172.

## Claims

1. A method to validate Next-generation sequencing (NGS) genotyping results of a panel of genes tested in a series of at least 2 patients **characterized in that** said validation is provided by SNP profiling assay, said method comprising the steps of:

   a) determining the genotype for a combination of at least 8 SNPs by an independent SNP profiling assay using the primary DNA samples used to obtain said NGS genotyping results, said NGS genotyping results including the genotype for said SNPs;
   b) comparing the SNPs genotypes obtained by said SNP profiling assay and NGS assay;
   c) validating or not NGS genotyping results based on said comparison, wherein:

      1) If there are not two patients from the series with identical SNP profiles, and said SNPs genotypes obtained by said SNP profiling assay and said NGS assay are identical, then NGS genotyping results are validated; and
      2) If two patients have identical SNP profiles but NGS genotyping results are distinct, a sequencing assay (e.g. Sanger sequencing) is further performed for these two patients, in order to validate their NGS genotyping results; and
      3) In other cases, NGS genotyping results are not validated and further validation is necessary;

   wherein said SNPs have the following features:

   i. they are not located in a repeated sequence of the genome;
   ii. they are biallelic;
   iii. the 60 bases flanking sequences at either side of the SNP site has a GC content < 70% and an AT content <70%,
   iv. they are not associated to a known pathology.

2. The method according to claim 1, wherein said SNPs further have one of the following features:

   v. they do not present significant linkage disequilibrium (LD) between each other;
   vi. they present a minor allele frequency (MAF) for a population comprised between 0.1 and 0.5, preferentially between 0.2 and 0.5, more preferentially between 0.25 and 0.5, even more preferentially between 0.275 and 0.5, preferentially between 0.3 and 0.5, even more preferentially between 0.325 and 0.5, even more preferentially between 0.35 and 0.5, even more preferentially between 0.375 and 0.5, even more preferentially between 0.4 and 0.5

preferentially said SNPs further have features v. and vi.

3. The method according to claim 1 or 2, wherein said SNPs are located in housekeeping genes.

4. The method according to any one of claims 1 to 3, wherein said combination of SNPs comprises at least one, preferentially at least 2, at least 8, more preferentially at least 12, even more preferentially at least 15, SNPs selected from rs11702450; rs843345; rs1058018; rs8017; rs3738494; rs1065483; rs2839181; rs11059924; rs2075144; rs6795772; rs456261; rs1131620; rs2231926; rs352169 and rs3739160.

5. The method according to any one of claims 1 to 4, wherein all of said SNPs are detected by allele-specific multiplex PCR with a specific set of primers, wherein said specific primers have the following features:

I. no additional SNP of frequency >5% is present within the said specific primers, and no additional SNP of frequency >1% is present within the 10 bases of the 3' end of the said specific primers;
II. their melting temperature is comprised between 62°C and 71°C, preferentially between 63°C and 68°C, more preferentially between 64 and 66°C, even more preferentially about 65°C (+/-1 °C);
III. they generate amplicons which do not contain any repeat, insertion or deletion frequent (>1%) polymorphism

wherein said specific set of primers comprises for each SNP the following triplet of primers:

a) 2 primers ("sense strand primers") hybridizing on the same DNA strand specifically at their 3' end to polymorphic nucleotide of alleles 1 and 2 of said SNP, respectively;
b) 1 primer specifically hybridizing to the opposite strand ("opposite strand primer").

6. The method according to claim 5, wherein specific primers of each pair consisting of a sense primer and an opposite primer intended for amplifying one allele of an SNP further have the following features:

IV. they do not form dimer at their 3'end with themselves, nor with each other, whose binding energy is below -3.6 Kcal/mol, preferentially -1.9 Kcal/mol;
V. they do not hybridize to the genome unspecifically;
VI. they generate amplicons with a size comprised between 90 and 500 base pairs.

7. The method according to claim 5 or 6, wherein said 2 sense strand primers comprise at least one base at the 3' end which is a Locked Nucleic Acid (LNA) base.

8. The method according to anyone of claims 5 to 7, wherein said sense strand primers or said opposite strand primers, preferably said opposite strand primers, have an additional GTTTCTT sequence added to their 5' end.

9. The method according to anyone of claims 5 to 8, wherein said pairs of primers intended to amplify one allele of an SNP are designed to generate amplicons of different sizes, and wherein:

IX. the sizes of amplicons related to the allele 1 and the allele 2 of $SNP^n$ differ by 2 to 5 base pairs, preferentially 3 base pairs; and
X. the sizes of amplicons related to the allele 2 of $SNP^n$ and the allele 1 of $SNP^{n+1}$ differ by 2 to 20 base pairs, preferentially 2 to 10 base pairs, more preferentially 3 to 8 base pairs, even more preferentially 4 to 6 base pairs; and
XI. said difference between the sizes of amplicons of allele 1 and allele 2 of each SNP is generated by adding bases to the 5' end of the sense strand primer hybridizing with allele 1 or 2 of the SNP, preferentially to allele 2 of the SNP.

10. The method according to any one of claims 5 to 9, wherein said sense strand primers or said opposite strand primers are labeled with a fluorochrome, such as 6-FAM, provided that, when the sense or opposite primers have a GTTTCTT sequence at their 5' end, the fluorochrome is attached to primer not comprising the GTTTCTT sequence at their 5' end.

**11.** The method according to any one of claims 5 to 10, wherein said combination of SNPs comprises, and more preferably consists of, all of rs11702450; rs843345; rs1058018; rs8017; rs3738494; rs1065483; rs2839181; rs11059924; rs2075144; rs6795772; rs456261; rs1131620; rs2231926; rs352169 and rs3739160, and said set of primers are selected in:

| | | | |
|---|---|---|---|
| 1 | SEQ IDNO 1 | MCM3AP_1323CL_F_Label | [LAB EL]CACAGCCATCCAGTGCAAGAA{C} |
| | SEQ IDNO 2 | MCM3AP_1323TL_F_Label | [LABEL]CAACACAGCCATCCAGTGCAAGAA{T} |
| | SEQ IDNO 3 | MCM3AP_ex2_q7_R | GTTTCTTAAGATGCGCTGCACTTTAGCAA |
| 2 | SEQ IDNO 4 | ABCF3_837-34TL_R_Label | [LABEL]AGAAACAGCAATTGGCCTAAGC{A} |
| | SEQ IDNO 5 | ABCF3_837-34CL_R_Label | [LABEL]ATGAGAAACAGCAATTGGCCTAAGC{G} |
| | SEQ IDNO 6 | ABCF3_q7_F | GTTTCTTATTCTCTTCCTCTTCCAGCCACA |
| 3 | SEQ IDNO 7 | UBE2Z_846CL_R_Label | [LABEL]GATCTTTGCAGGCCACCTC{G} |
| | SEQ IDNO 8 | UBE2Z_846TL_R_Label | [LABEL]GATGATCTTTGCAGGCCACCTC{A} |
| | SEQ IDNO 9 | UBE2Z_q7_F | GTTTCTTTGACCTGTACCCCTGGGTTTCT |
| 4 | SEQ IDNO 10 | TCEB2_386GL_R_Label | [LABEL]GGCTCCAGCTTGTGTTTCTG{C} |
| | SEQ IDNO 11 | TCEB2_386AL_R_Label | [LABEL]TTGGGCTCCAGCTTGTGTTTCTG{T} |
| | SEQ IDNO 12 | TCEB2_q7_F | GTTTCTTCCAGCCTCAGGGACAAGAGATT |
| 5 | SEQ IDNO 13 | PPIH_132-40CL_F_Label | [LABEL]GAGGCGCTCACGACTGTGA{C} |
| | SEQ IDNO 14 | PPIH_132-40TL_F_Label | [LAB EL]CAAGAGGCGCTCACGACTGTGA{T} |
| | SEQ IDNO 15 | PPIH_q7_R | GTTTCTTACCCCTCTGGAGCAGGCAA |

(continued)

| | | | |
|---|---|---|---|
| 6 | SEQ IDNO 16 | RABEP1_2457GL2_F_Label | [LAB EL]GATGTCAGTGAGCAAGTCCAGA{GG} |
| | SEQ IDNO 17 | RABEP1_2457AL_F_Label | [LABEL]AGAGATGTCAGTGAGCAAGTCCAGAG{A} |
| | SEQ IDNO 18 | RABEP1_q7_R | GTTTCTTCAGTGGTCAAGTCAGGGATCGG |
| 7 | SEQ IDNO 19 | MCM3AP_2931TL_R_Label | [LABEL]TTGAAGCTGCACACAGGGGT{A} |
| | SEQ IDNO 20 | MCM3AP_2931CL_R_Label | [LABEL]TCATTGAAGCTGCACACAGGGGT{G} |
| | SEQ IDNO 21 | MCM3AP_q7_F | GTTTCTTGTCTGCATTCCTGGAACCAGAG |
| 8 | SEQ IDNO 22 | SLC15A4_1245GL_F_Label | [LAB EL]GCATGTTCTTTGTCATGTGCTC{G} |
| | SEQ IDNO 23 | SLC15A4_1245AL_F_Label | [LABEL]AACGCATGTTCTTTGTCATGTGCTC{A} |
| | SEQ IDNO 24 | SLC15A4_q7_R | GTTTCTTTTTACAGACATGCACTTCCTGAACAAC |
| 9 | SEQ IDNO 25 | PPP5C_363+40GL_R_Label | [LABEL]GCCCAGCCCTCAGTATCTG{C} |
| | SEQ IDNO 26 | PPP5C_363+40AL_R_Label | [LABEL]TTCGCCCAGCCCTCAGTATCTG{T} |
| | SEQ IDNO 27 | PPP5C_q7_F | GTTTCTTCCATTGAGCTGGACAAGAAGTACATC |
| 10 | SEQ IDNO 28 | USP4_230-20GL_F_Label | [LABEL]TCTGGGGTAAAGAGCAGTGACTTAT{G} |
| | SEQ IDNO 29 | USP4_230-20AL_F_Label | [LABEL]ACATCTGGGGTAAAGAGCAGTGACTTAT{A} |
| | SEQ IDNO 30 | USP4_q7_R | GTTTCTTCGATGGGTTGCTGGCCTTCTA |
| 11 | SEQ IDNO 31 | PFDN6_261-50GL_R_Label | [LABEL]CAAGCAGAAAGGGAGAAATTAGTAGGACT{C} |

(continued)

| | | | |
|---|---|---|---|
| | SEQ IDNO 32 | PFDN6_261-50AL_R_Label | [LABEL]TGACAAGCAGAAAGGGAGAAATTAGTAGGACT{T} |
| | SEQ IDNO 33 | PFDN6_q7_F | GTTTCTTAACCATTGCAGAACAGCTCTCCAT |
| 12 | SEQ IDNO 34 | LTBP4_2359AL_R_Label | [LABEL]CGCACTCGGAGCCAGCAG{T} |
| | SEQ IDNO 35 | LTBP4_2359GL2_R_Label | [LAB EL]TGACGCACTCGGAGCCAGCA{GC} |
| | SEQ IDNO 36 | LTBP4_q7_F | GTTTCTTTGATGGCCATGGGAATGGAT |
| 13 | SEQ IDNO 37 | PPP4R2_420-1015AL_R_Label | [LAB EL]TTATCACTTGATCCAGCCGCAA{T} |
| | SEQ IDNO 38 | PPP4R2_420-1015GL2_R_Label | [LABEL]CAGTTATCACTTGATCCAGCCGCA{AC} |
| | SEQ IDNO 39 | PPP4R2_q7_F | GTTTCTTGATGGGTTACACCAGGCATTACTGA |
| 14 | SEQ IDNO 40 | ALAS1_427+12GL_F_Label | [LABEL]CCGTGAGGAAAGGTAAGAGATGA{G} |
| | SEQ IDNO 41 | ALAS1_427+12AL_F_Label | [LABEL]ACTCCGTGAGGAAAGGTAAGAGATGA{A} |
| | SEQ IDNO 42 | ALAS1_q7_R | GTTTCTTCGCACCAGAAAGAAAGTCCCA |
| 15 | SEQ IDNO 43 | MRPS9_135+31CL_F_Label | [LABEL]GGAAGACTGGAAGCGGCTTA{C} |
| | SEQ IDNO 44 | MRPS9_135+31TL_F_Label | [LABEL]CATGGAAGACTGGAAGCGGCTTA{T} |
| | SEQ IDNO 45 | MRPS9_q7_R | GTTTCTTAGGTCGCTCCACTTCTACCTTCA |

wherein bases in braces are LNA modified bases; [LABEL] is the 5' modification of the primer.

**12.** The method according to claim 9 or 11, wherein said SNPs are detected by determining the size of said amplicons generated by allele-specific multiplex PCR, preferably by method for separation of DNA based on size, such as capillary electrophoresis.

**13.** The method according to claim 12, wherein said SNP profiling assay in said step b) is automated with a software recognizing the said labeled multiplex PCR products.

**14.** The method according to any one of claims 1 to 13, wherein said NGS is target capture NGS or amplicon NGS.

**15.** A kit for detection of a combination of at least 8 SNPs as defined in claim 4, comprising primers as defined in any one of claims 5 to 11, said kit preferably further comprising:

- PCR multiplex reagents; and/or
- NGS oligonucleotide probes or primers designed to capture or amplify sequences comprising said at least 8 SNPs.

**16.** Use of the kit according to claim 15 in a method to validate NGS genotyping results of a panel of genes tested in series of at least 2 patients, according to any one of claims 1 to 14.

**17.** A method for detecting polymorphisms in the DNA of a patient, comprising performing, preferentially in parallel, the two following steps:

a) detecting polymorphisms by NGS assay, and
b) validating NGS genotyping results using the method according to any one of claims 1 to 14.

**Patentansprüche**

**1.** Verfahren zur Validierung von Next-Generation-Sequencing-Genotypisierungsergebnissen (NGS-Genotypisierungsergebnissen) eines Panels von Genen, die in einer Reihe von mindestens 2 Patienten getestet wurden, **dadurch gekennzeichnet, dass** die Validierung durch einen SNP-Profilierungsassay bereitgestellt wird, wobei das Verfahren die Schritte umfasst:

a) Bestimmen des Genotyps für eine Kombination von mindestens 8 SNP durch einen unabhängigen SNP-Profilierungsassay unter Verwendung der primären DNA-Proben, die zum Erhalten der NGS-Genotypisierungsergebnisse verwendet wurden, wobei die NGS-Genotypisierungsergebnisse den Genotyp für die SNP beinhalten;
b) Vergleichen der SNP-Genotypen, die durch den SNP-Profilierungsassay und den NGS-Assay erhalten wurden;
c) Validieren der NGS-Genotypisierungsergebnisse auf der Basis des Vergleichs oder auch nicht, wobei:

1) Wenn keine zwei Patienten von der Reihe mit identischen SNP-Profilen vorliegen und die SNP-Genotypen, die durch den SNP-Profilierungsassay und den NGS-Assay erhalten wurden, identisch sind, die NGS-Genotypisierungsergebnisse validiert werden; und
2) Wenn zwei Patienten identische SNP-Profile aufweisen, die NGS-Genotypisierungsergebnisse unterschiedlich sind, ferner ein Sequenzierungsassay (z. B. Sanger-Sequenzierung) für diese zwei Patienten durchgeführt wird, um ihre NGS-Genotypisierungsergebnisse zu validieren; und
3) In anderen Fällen die NGS-Genotypisierungsergebnisse nicht validiert werden und eine weitere Validierung erforderlich ist;

wobei die SNP die folgenden Merkmale aufweisen:

i. sie befinden sich nicht in einer wiederholten Sequenz des Genoms;
ii. sie sind biallelisch;
iii. die flankierenden 60-Basen-Sequenzen auf beiden Seiten des SNP-Orts weisen einen GC-Gehalt < 70% und einen AT-Gehalt < 70% auf,
iv. sie sind nicht mit einer bekannten Pathologie assoziiert.

**2.** Verfahren nach Anspruch 1, wobei die SNP ferner eines der folgenden Merkmale aufweisen:

v. sie präsentieren kein signifikantes Kopplungsungleichgewicht (LD) zwischen einander;
vi. sie präsentieren eine Häufigkeit des selteneren Allels (MAF) für eine Population, die zwischen 0,1 und 0,5, vorzugsweise zwischen 0,2 und 0,5, mehr bevorzugt zwischen 0,25 und 0,5, noch mehr bevorzugt zwischen 0,275 und 0,5, vorzugsweise zwischen 0,3 und 0,5, noch mehr bevorzugt zwischen 0,325 und 0,5, noch mehr bevorzugt zwischen 0,35 und 0,5, noch mehr bevorzugt zwischen 0,375 und 0,5, noch mehr bevorzugt zwischen 0,4 und 0,5 liegt,

vorzugsweise wobei die SNP ferner die Merkmale v. und vi. aufweisen.

**3.** Verfahren nach Anspruch 1 oder 2, wobei die SNP sich in Haushaltsgenen befinden.

**4.** Verfahren nach einem der Ansprüche 1 bis 3, wobei die Kombination von SNP mindestens einen, vorzugsweise mindestens 2, mindestens 8, mehr bevorzugt mindestens 12, noch mehr bevorzugt mindestens 15 SNP umfasst, die aus rs11702450; rs843345; rs1058018; rs8017; rs3738494; rs1065483; rs2839181; rs11059924; rs2075144; rs6795772; rs456261; rs1131620; rs2231926; rs352169 und rs3739160 ausgewählt sind.

**5.** Verfahren nach einem der Ansprüche 1 bis 4, wobei alle der SNP durch eine allelspezifische Multiplex-PCR mit einem spezifischen Satz von Primern nachgewiesen werden, wobei die spezifischen Primer die folgenden Merkmale aufweisen:

I. kein zusätzlicher SNP mit einer Häufigkeit > 5% liegt innerhalb der spezifischen Primer vor und kein zusätzlicher Primer mit einer Häufigkeit > 1% liegt innerhalb der 10 Basen des 3'-Endes der spezifischen Primer vor;
II. ihre Schmelztemperatur liegt zwischen 62°C und 71°C, vorzugsweise zwischen 63°C und 68°C, mehr bevorzugt zwischen 64 und 66°C, noch mehr bevorzugt bei etwa 65°C (+/- 1°C);
III. sie erzeugen Amplikone, die keinen wiederholungs-, insertions- oder deletionshäufigen (> 1%) Polymorphismus enthalten,

wobei der spezifische Satz von Primern für jeden SNP das folgende Triplett von Primern umfasst:

a) 2 Primer ("Sense-Strang-Primer"), die an demselben DNA-Strang, spezifisch an ihrem 3'-Ende an ein polymorphes Nukleotid von Allel 1 bzw. 2 des SNP hybridisieren;
b) 1 Primer, der spezifisch an den entgegengesetzten Strang ("Entgegengesetzter-Strang-Primer") hybridisiert.

**6.** Verfahren nach Anspruch 5, wobei spezifische Primer jedes Paars, das aus einem Sense-Primer und einem entgegengesetzten Primer besteht, die zum Amplifizieren eines Allels eines SNP vorgesehen sind, ferner die folgenden Merkmale aufweisen:

IV. sie bilden kein Dimer an ihrem 3'-Ende und mit sich selbst noch miteinander, dessen Bindungsenergie weniger als - 3,6 kcal/mol, vorzugsweise -1,9 kcal/mol beträgt;
V. sie hybridisieren nicht unspezifisch an das Genom;
VI. sie erzeugen Amplikone mit einer Größe, die zwischen 90 und 500 Basenpaaren liegt.

**7.** Verfahren nach Anspruch 5 oder 6, wobei die 2 Sense-Strang-Primer mindestens eine Base am 3'-Ende umfassen, die eine gesperrte Nukleinsäurenbase (LNA-Base) ist.

**8.** Verfahren nach einem der Ansprüche 5 bis 7, wobei die Sense-Strang-Primer oder die Entgegengesetzter-Strang-Primer, vorzugsweise die Entgegengesetzter-Strang-Primer eine zusätzliche GTTTCTT-Sequenz aufweisen, die an ihr 5'-Ende hinzugefügt ist.

**9.** Verfahren nach einem der Ansprüche 5 bis 8, wobei die Paare von Primern, die zum Amplifizieren eines Allels eines SNP vorgesehen sind, dazu entworfen sind, Amplikone mit unterschiedlichen Größen zu erzeugen, und wobei:

IX. die Größen von Amplikonen, die mit dem Allel 1 und dem Allel 2 von $SNP^n$ in Zusammenhang stehen, sich um 2 bis 5 Basenpaare, vorzugsweise 3 Basenpaare unterscheiden; und
X. die Größen von Amplikonen, die mit dem Allel 2 von $SNP^n$ und dem Allel 1 von $SNP^{n+1}$ in Zusammenhang stehen, sich um 2 bis 20 Basenpaare, vorzugsweise 2 bis 10 Basenpaare, mehr bevorzugt 3 bis 8 Basenpaare, noch mehr bevorzugt 4 bis 6 Basenpaare unterscheiden; und
XI. der Unterschied zwischen den Größen von Amplikonen von Allel 1 und Allel 2 jedes SNP durch Hinzufügen von Basen an das 5'-Ende des Sense-Strang-Primers, der mit Allel 1 oder 2 des SNP, vorzugsweise an Allel 2 des SNP hybridisiert, erzeugt wird.

**10.** Verfahren nach einem der Ansprüche 5 bis 9, wobei die Sense-Strang-Primer oder die Entgegengesetzter-Strang-Primer mit einem Fluorochrom, wie 6-FAM, markiert werden, vorausgesetzt, dass, wenn die Sense- oder entgegengesetzten Primer eine GTTTCTT-Sequenz an ihrem 5'-Ende aufweisen, das Fluorochrom an einen Primer angelagert wird, der nicht die GTTTCTT-Sequenz an seinem 5'-Ende umfasst.

**11.** Verfahren nach einem der Ansprüche 5 bis 10, wobei die Kombination von SNP alle von rs11702450; rs843345; rs1058018; rs8017; rs3738494; rs1065483; rs2839181; rs11059924; rs2075144; rs6795772; rs456261; rs1131620; rs2231926; rs352169 und rs3739160 umfasst und mehr bevorzugt daraus besteht und der Satz von Primer ausgewählt ist in:

| | | | |
|---|---|---|---|
| 1 | SEQ ID Nr. 1 | MCM3AP_1323CL_F_Label | [MARKER]CACAGCCATCCAGTGCAAGAA{C} |
| | SEQ ID Nr. 2 | MCM3AP_1323TL_F_Label | [MARKER]CAACACAGCCATCCAGTGCAAGAA{T} |
| | SEQ ID Nr. 3 | MCM3AP_ex2_q7_R | GTTTCTTAAGATGCGCTGCACTTTAGCAA |
| 2 | SEQ ID Nr. 4 | ABCF3_837-34TL_R_Label | [MARKER]AGAAACAGCAATTGGCCTAAGC{A} |
| | SEQ ID Nr. 5 | ABCF3_837-34CL_R_Label | [MARKER]ATGAGAAACAGCAATTGGCCTAAGC{G} |
| | SEQ ID Nr. 6 | ABCF3_q7_F | GTTTCTTATTCTCTTCCTCTTCCAGCCACA |
| 3 | SEQ ID Nr. 7 | UBE2Z_846CL_R_Label | [MARKER]GATCTTTGCAGGCCACCTC{G} |
| | SEQ ID Nr. 8 | UBE2Z_846TL_R_Label | [MARKER]GATGATCTTTGCAGGCCACCTC{A} |
| | SEQ ID Nr. 9 | UBE2Z_q7_F | GTTTCTTTGACCTGTACCCCTGGGTTTCT |
| 4 | SEQ ID Nr. 10 | TCEB2_386GL_R_Label | [MARKER]GGCTCCAGCTTGTGTTTCTG{C} |
| | SEQ ID Nr. 11 | TCEB2_386AL_R_Label | [MARKER]TTGGGCTCCAGCTTGTGTTTCTG{T} |
| | SEQ ID Nr. 12 | TCEB2_q7_F | GTTTCTTCCAGCCTCAGGGACAAGAGATT |
| 5 | SEQ ID Nr. 13 | PPIH_132-40CL_F_Label | [MARKER]GAGGCGCTCACGACTGTGA{C} |

(fortgesetzt)

| | | | |
|---|---|---|---|
| | SEQ ID Nr. 14 | PPIH_132-40TL_F_Label | [MARKER]CAAGAGGCGCTCACGACTGTGA{T} |
| | SEQ ID Nr. 15 | PPIH_q7_R | GTTTCTTACCCCTCTGGAGCAGGCAA |
| 6 | SEQ ID Nr. 16 | RABEP1_2457GL2_F_Label | [MARKER]GATGTCAGTGAGCAAGTCCAGA{GG} |
| | SEQ ID Nr. 17 | RABEP1_2457AL_F_Label | [MARKER]AGAGATGTCAGTGAGCAAGTCCAGAG{A} |
| | SEQ ID Nr. 18 | RABEP1_q7_R | GTTTCTTCAGTGGTCAAGTCAGGGATCGG |
| 7 | SEQ ID Nr. 19 | MCM3AP_2931TL_R_Label | [MARKER]TTGAAGCTGCACACAGGGGT{A} |
| | SEQ ID Nr. 20 | MCM3AP_2931CL_R_Label | [MARKER]TCATTGAAGCTGCACACAGGGGT{G} |
| | SEQ ID Nr. 21 | MCM3AP_q7_F | GTTTCTTGTCTGCATTCCTGGAACCAGAG |
| 8 | SEQ ID Nr. 22 | SLC15A4_1245GL_F_Label | [MARKER]GCATGTTCTTTGTCATGTGCTC{G} |
| | SEQ ID Nr. 23 | SLC15A4_1245AL_F_Label | [MARKER]AACGCATGTTCTTTGTCATGTGCTC{A} |
| | SEQ ID Nr. 24 | SLC15A4_q7_R | GTTTCTTTTTACAGACATGCACTTCCTGAACAAC |
| 9 | SEQ ID Nr. 25 | PPP5C_363+40GL_R_Label | [MARKER]GCCCAGCCCTCAGTATCTG{C} |
| | SEQ ID Nr. 26 | PPP5C_363+40AL_R_Label | [MARKER]TTCGCCCAGCCCTCAGTATCTG{T} |
| | SEQ ID Nr. 27 | PPP5C_q7_F | GTTTCTTCCATTGAGCTGGACAAGAAGTACATC |
| 10 | SEQ ID Nr. 28 | USP4_230-20GL_F_Label | [MARKER]TCTGGGGTAAAGAGCAGTGACTTAT{G} |
| | SEQ ID Nr. 29 | USP4_230-20AL_F_Label | [MARKER]ACATCTGGGGTAAAGAGCAGTGACTTAT{A} |

(fortgesetzt)

| | | | |
|---|---|---|---|
| | SEQ ID Nr. 30 | USP4_q7_R | GTTTCTTCGATGGGTTGCTGGCCTTCTA |
| 11 | SEQ ID Nr. 31 | PFDN6_261-50GL_R_Label | [MARKER]CAAGCAGAAAGGGAGAAATTAGTAGGACT{C} |
| | SEQ ID Nr. 32 | PFDN6_261-50AL_R_Label | [MARKER]TGACAAGCAGAAAGGGAGAAATTAGTAGGACT{T} |
| | SEQ ID Nr. 33 | PFDN6_q7_F | GTTTCTTAACCATTGCAGAACAGCTCTCCAT |
| 12 | SEQ ID Nr. 34 | LTBP4_2359AL_R_Label | [MARKER]CGCACTCGGAGCCAGCAG{T} |
| | SEQ ID Nr. 35 | LTBP4_2359GL2_R_Label | [MARKER]TGACGCACTCGGAGCCAGCA{GC} |
| | SEQ ID Nr. 36 | LTBP4_q7_F | GTTTCTTTGATGGCCATGGGAATGGAT |
| 13 | SEQ ID Nr. 37 | PPP4R2_420-1015AL_R_Label | [MARKER]TTATCACTTGATCCAGCCGCAA{T} |
| | SEQ ID Nr. 38 | PPP4R2_420-1015GL2_R_Label | [MARKER]CAGTTATCACTTGATCCAGCCGCA{AC} |
| | SEQ ID Nr. 39 | PPP4R2_q7_F | GTTTCTTGATGGGTTACACCAGGCATTACTGA |
| 14 | SEQ ID Nr. 40 | ALAS1_427+12GL_F_Label | [MARKER]CCGTGAGGAAAGGTAAGAGATGA{G} |
| | SEQ ID Nr. 41 | ALAS1_427+12AL_F_Label | [MARKER]ACTCCGTGAGGAAAGGTAAGAGATGA{A} |
| | SEQ ID Nr. 42 | ALAS1_q7_R | GTTTCTTCGCACCAGAAAGAAAGTCCCA |
| 15 | SEQ ID Nr. 43 | MRPS9_135+31CL_F_Label | [MARKER]GGAAGACTGGAAGCGGCTTA{C} |
| | SEQ ID Nr. 44 | MRPS9_135+31TL_F_Label | [MARKER]CATGGAAGACTGGAAGCGGCTTA{T} |
| | SEQ ID Nr. 45 | MRPS9_q7_R | GTTTCTTAGGTCGCTCCACTTCTACCTTCA |

wobei Basen in Klammern LNA-modifizierte Basen sind; [MARKER] die 5'-Modifikation des Primers ist.

**12.** Verfahren nach Anspruch 9 oder 11, wobei die SNP durch Bestimmen der Größe der Amplikone, die durch eine allelspezifische Multiplex-PCR erzeugt werden, vorzugsweise durch ein Verfahren zur Trennung von DNA auf der Basis der Größe, wie Kapillar-Elektrophorese, nachgewiesen werden.

**13.** Verfahren nach Anspruch 12, wobei der SNP-Profilierungsassay im Schritt b) mit einer Software automatisiert wird, die die markierten Multiplex-PCR-Produkte erkennt.

**14.** Verfahren nach einem der Ansprüche 1 bis 13, wobei die NGS eine Target-Capture-NGS oder eine Amplikon-NGS ist.

**15.** Kit zum Nachweis einer Kombination von mindestens 8 SNP, wie in Anspruch 4 definiert, umfassend Primer, wie in einem der Ansprüche 5 bis 11 definiert, wobei das Kit vorzugsweise ferner umfasst:

- PCR-Multiplex-Reagentien; und/oder
- NGS-Oligonukleotid-Sonden oder -Primer, die dazu entworfen sind, Sequenzen, die mindestens 8 SNP umfasst, einzufangen oder zu amplifizieren.

**16.** Verwendung des Kits nach Anspruch 15 in einem Verfahren zur Validierung von NGS-Genotypisierungsergebnissen eines Panels von Genen, die in einer Reihe von mindestens 2 Patienten getestet wurden, nach einem der Ansprüche 1 bis 14.

**17.** Verfahren zum Nachweis von Polymorphismen in der DNA eines Patienten, umfassend ein Durchführen, vorzugsweise parallel, der zwei folgenden Schritte:

a) Nachweisen von Polymorphismen durch einen NGS-Assay, und
b) Validieren von NGS-Genotypisierungsergebnissen unter Verwendung des Verfahrens nach einem der Ansprüche 1 bis 14.

## Revendications

**1.** Procédé pour valider des résultats de génotypage par séquençage de nouvelle génération (NGS) d'un panel de gènes testés dans une série d'au moins 2 patients, **caractérisé en ce que** ladite validation est fournie par un test de profilage de SNP, ledit procédé comprenant les étapes de :

a) détermination du génotype pour une combinaison d'au moins 8 SNP par un test de profilage de SNP indépendants utilisant les échantillons d'ADN primaires utilisés pour l'obtention desdits résultats de génotypage par NGS, lesdits résultats de génotypage par NGS comprenant le génotype pour lesdits SNP ;
b) comparaison des génotypes de SNP obtenus par ledit test de profilage de SNP et le test par NGS ;
c) validation ou pas des résultats de génotypage par NGS sur la base de ladite comparaison, dans laquelle :

1) S'il n'y a pas deux patients de la série ayant des profils de SNP identiques, et lesdits génotypes de SNP obtenus par ledit test de profilage de SNP et ledit test par NGS sont identiques, alors les résultats de génotypage par NGS sont validés ; et
2) Si deux patients ont des profils de SNP identiques mais les résultats de génotypage par NGS sont distincts, un test par séquençage (par exemple un séquençage de Sanger) est en outre réalisé pour ces deux patients, afin que leurs résultats de génotypage par NGS soient validés ; et
3) Dans les autres cas, les résultats de génotypage par NGS ne sont pas validés et une validation ultérieure est nécessaire ;

dans lequel lesdits SNP ont les caractéristiques suivantes :

i. ils ne sont pas situés dans une séquence répétée du génome ;
ii. ils sont bialléliques ;
iii. les séquences flanquantes de 60 bases sur l'un ou l'autre côté du site de SNP ont une teneur en GC < 70 % et une teneur en AT < 70 %,
iv. ils ne sont pas associés à une pathologie connue.

**2.** Procédé selon la revendication 1, dans lequel lesdits SNP ont en outre l'une des caractéristiques suivantes :

v. ils ne présentent pas de déséquilibre de liaison (LD) significatif les uns avec les autres ;
vi. ils présentent une fréquence d'allèles mineurs (MAF) pour une population comprise entre 0,1 et 0,5, de préférence entre 0,2 et 0,5, mieux encore entre 0,25 et 0,5, plus particulièrement entre 0,275 et 0,5, de préférence entre 0,3 et 0,5, mieux encore entre 0,325 et 0,5, plus particulièrement entre 0,35 et 0,5, plus particulièrement entre 0,375 et 0,5, plus particulièrement entre 0,4 et 0,5

de préférence lesdits SNP ont en outre les caractéristiques v. et vi.

**3.** Procédé selon la revendication 1 ou 2, dans lequel lesdits SNP sont situés dans des gènes domestiques.

**4.** Procédé selon l'une quelconque des revendications 1 à 3, dans lequel ladite combinaison de SNP comprend au moins un, de préférence au moins 2, au moins 8, mieux encore au moins 12, plus particulièrement au moins 15 SNP choisis parmi rs11702450 ; rs843345 ; rs1058018 ; rs8017 ; rs3738494 ; rs1065483 ; rs2839181 ; rs11059924 ; rs2075144 ; rs6795772 ; rs456261 ; rs1131620 ; rs2231926 ; rs352169 et rs3739160.

**5.** Procédé selon l'une quelconque des revendications 1 à 4, dans lequel tous lesdits SNP sont détectés par PCR multiplex à spécificité d'allèle avec un jeu d'amorces spécifiques, dans lequel lesdites amorces spécifiques ont les caractéristiques suivantes :

I. aucun SNP additionnel avec une fréquence > 5 % n'est présent dans lesdites amorces spécifiques, et aucun SNP additionnel avec une fréquence > 1 % n'est présent dans les 10 bases de l'extrémité 3' desdites amorces spécifiques ;
II. leur point de fusion est compris entre 62 °C et 71 °C, de préférence entre 63 °C et 68 °C, mieux encore entre 64 et 66 °C, plus particulièrement d'environ 65 °C (± 1 °C) ;
III. elles génèrent des amplicons qui ne contiennent aucun polymorphisme de répétition, insertion ou délétion fréquente (> 1 %)

dans lequel ledit jeu d'amorces spécifiques comprend pour chaque SNP le triplet suivant d'amorces :

a) 2 amorces (« amorces de brin sens ») s'hybridant sur le même brin d'ADN spécifiquement à leur extrémité 3' à un polynucléotide polymorphique d'allèles 1 et 2 dudit SNP, respectivement ;
b) 1 amorce s'hybridant spécifiquement au brin opposé (« amorce de brin opposé »).

**6.** Procédé selon la revendication 5, dans lequel des amorces spécifiques de chaque paire constituées d'une amorce sens et d'une amorce opposée destinées à l'amplification d'un allèle donné d'un SNP ont en outre les caractéristiques suivantes :

IV. elles ne forment pas de dimère à leur extrémité 3' ni avec elles-mêmes ni entre elles, et leur énergie de liaison est inférieure à -3,6 kcal/mol, de préférence -1,9 kcal/mol ;
V. elles ne s'hybrident pas au génome de façon non spécifique ;
VI. elles génèrent des amplicons ayant une taille comprise entre 90 et 500 paires de bases.

**7.** Procédé selon la revendication 5 ou 6, dans lequel 2 amorces de brin sens comprennent au moins une base à l'extrémité 3' qui est une base de type acide nucléique verrouillé (LNA).

**8.** Procédé selon l'une quelconque des revendications 5 à 7, dans lequel lesdites amorces de brin sens ou lesdites amorces de brin opposé, de préférence lesdites amorces de brin opposé, ont une séquence GTTTCTT ajoutée à leur extrémité 5'.

**9.** Procédé selon l'une quelconque des revendications 5 à 8, dans lequel lesdites paires d'amorces destinées à amplifier un allèle donné d'un SNP sont conçues pour générer des amplicons de différentes tailles, et dans lequel

IX. les tailles des amplicons associés à l'allèle 1 et à l'allèle 2 du $SNP^n$ diffèrent de 2 à 5 paires de bases, de préférence 3 paires de bases ; et
X. les tailles des amplicons associés à l'allèle 2 du $SNP^n$ et à l'allèle 1 du $SNP^{n+1}$ diffèrent de 2 à 20 paires de bases, de préférence de 2 à 10 paires de bases, mieux encore de 3 à 8 paires de bases, plus particulièrement de 4 à 6 paires de bases ; et

XI. ladite différence entre les tailles des amplicons d'allèle 1 et d'allèle 2 de chaque SNP est générée par l'addition de bases à l'extrémité 5' de l'amorce de brin sens s'hybridant avec l'allèle 1 ou 2 du SNP, de préférence l'allèle 2 du SNP.

**10.** Procédé selon l'une quelconque des revendications 5 à 9, dans lequel lesdites amorces de brin sens ou lesdites amorces de brin opposé sont marquées par un fluorochrome, tel que 6-FAM, sous réserve que, lorsque les amorces sens ou opposées ont une séquence GTTTCTT à leur extrémité 5', le fluorochrome soit rattaché à l'amorce ne comprenant pas la séquence GTTTCTT à son extrémité 5'.

**11.** Procédé selon l'une quelconque des revendications 5 à 10, dans lequel ladite combinaison de SNP comprend, et mieux encore est constituée de, la totalité parmi rs11702450; rs843345 ; rs1058018 ; rs8017 ; rs3738494 ; rs1065483 ; rs2839181 ; rs11059924; rs2075144 ; rs6795772 ; rs456261 ; rs1131620 ; rs2231926 ; rs352169 et rs3739160, et ledit jeu d'amorces est choisi parmi :

| | | | |
|---|---|---|---|
| 1 | SEQ IDNO 1 | MCM3AP_1323CL_F_Label | [LABEL]CACAGCCATCCAGTGCAAGAA{C} |
| | SEQ IDNO 2 | MCM3AP_1323TL_F_Label | [LABEL]CAACACAGCCATCCAGTGCAAGAA{T} |
| | SEQ IDNO 3 | MCM3AP_ex2_q7_R | GTTTCTTAAGATGCGCTGCACTTTAGCAA |
| 2 | SEQ IDNO 4 | ABCF3_837-34TL_R_Label | [LABEL]AGAAACAGCAATTGGCCTAAGC{A} |
| | SEQ IDNO 5 | ABCF3_837-34CL_R_Label | [LABEL]ATGAGAAACAGCAATTGGCCTAAGC{G} |
| | SEQ IDNO 6 | ABCF3_q7_F | GTTTCTTATTCTCTTCCTCTTCCAGCCACA |
| 3 | SEQ IDNO 7 | UBE2Z_846CL_R_Label | [LABEL]GATCTTTGCAGGCCACCTC{G} |
| | SEQ IDNO 8 | UBE2Z_846TL_R_Label | [LABEL]GATGATCTTTGCAGGCCACCTC{A} |
| | SEQ IDNO 9 | UBE2Z_q7_F | GTTTCTTTGACCTGTACCCCTGGGTTTCT |
| 4 | SEQ IDNO 10 | TCEB2_386GL_R_Label | [LABEL]GGCTCCAGCTTGTGTTTCTG{C} |
| | SEQ IDNO 11 | TCEB2_386AL_R_Label | [LABEL]TTGGGCTCCAGCTTGTGTTTCTG{T} |
| | SEQ IDNO 12 | TCEB2_q7_F | GTTTCTTCCAGCCTCAGGGACAAGAGATT |

(suite)

| | | | |
|---|---|---|---|
| 5 | SEQ IDNO 13 | PPIH_132-40CL_F_Label | [LABEL]GAGGCGCTCACGACTGTGA{C} |
| | SEQ IDNO 14 | PPIH_132-40TL_F_Label | [LABEL]CAAGAGGCGCTCACGACTGTGA{T} |
| | SEQ IDNO 15 | PPIH_q7_R | GTTTCTTACCCCTCTGGAGCAGGCAA |
| 6 | SEQ IDNO 16 | RABEP1_2457GL2_F_Label | [LABEL]GATGTCAGTGAGCAAGTCCAGA{GG} |
| | SEQ IDNO 17 | RABEP1_2457AL_F_Label | [LABEL]AGAGATGTCAGTGAGCAAGTCCAGAG{A} |
| | SEQ IDNO 18 | RABEP1_q7_R | GTTTCTTCAGTGGTCAAGTCAGGGATCGG |
| 7 | SEQ IDNO 19 | MCM3AP_2931TL_R_Label | [LABEL]TTGAAGCTGCACACAGGGGT{A} |
| | SEQ IDNO 20 | MCM3AP_2931 CL_R_Label | [LABEL]TCATTGAAGCTGCACACAGGGGT{G} |
| | SEQ IDNO 21 | MCM3AP_q7_F | GTTTCTTGTCTGCATTCCTGGAACCAGAG |
| 8 | SEQ IDNO 22 | SLC15A4_1245GL_F_Label | [LABEL]GCATGTTCTTTGTCATGTGCTC{G} |
| | SEQ IDNO 23 | SLC15A4_1245AL_F_Label | [LABEL]AACGCATGTTCTTTGTCATGTGCTC{A} |
| | SEQ IDNO 24 | SLC15A4_q7_R | GTTTCTTTTTACAGACATGCACTTCCTGAACAAC |
| 9 | SEQ IDNO 25 | PPP5C_363+40GL_R_Label | [LABEL]GCCCAGCCCTCAGTATCTG{C} |
| | SEQ IDNO 26 | PPP5C_363+40AL_R_Label | [LABEL]TTCGCCCAGCCCTCAGTATCTG{T} |
| | SEQ IDNO 27 | PPP5C_q7_F | GTTTCTTCCATTGAGCTGGACAAGAAGTACATC |
| 10 | SEQ IDNO 28 | USP4_230-20GL_F_Label | [LABEL]TCTGGGGTAAAGAGCAGTGACTTAT{G} |

(suite)

| | | | |
|---|---|---|---|
| | SEQ IDNO 29 | USP4_230-20AL_F_Label | [LABEL]ACATCTGGGGTAAAGAGCAGTGACTTAT{A} |
| | SEQ IDNO 30 | USP4_q7_R | GTTTCTTCGATGGGTTGCTGGCCTTCTA |
| 11 | SEQ IDNO 31 | PFDN6_261-50GL_R_Label | [LABEL]CAAGCAGAAAGGGAGAAATTAGTAGGACT{C} |
| | SEQ IDNO 32 | PFDN6_261-50AL_R_Label | [LABEL]TGACAAGCAGAAAGGGAGAAATTAGTAGGACT {T} |
| | SEQ IDNO 33 | PFDN6_q7_F | GTTTCTTAACCATTGCAGAACAGCTCTCCAT |
| 12 | SEQ IDNO 34 | LTBP4_2359AL_R_Label | [LABEL]CGCACTCGGAGCCAGCAG{T} |
| | SEQ IDNO 35 | LTBP4_2359GL2_R_Label | [LABEL]TGACGCACTCGGAGCCAGCA{GC} |
| | SEQ IDNO 36 | LTBP4_q7_F | GTTTCTTTGATGGCCATGGGAATGGAT |
| 13 | SEQ IDNO 37 | PPP4R2_420-1015AL_R_Label | [LABEL]TTATCACTTGATCCAGCCGCAA{T} |
| | SEQ IDNO 38 | PPP4R2_420-1015GL2_R_Label | [LABEL]CAGTTATCACTTGATCCAGCCGCA{AC} |
| | SEQ IDNO 39 | PPP4R2_q7_F | GTTTCTTGATGGGTTACACCAGGCATTACTGA |
| 14 | SEQ IDNO 40 | ALAS1_427+12GL_F_Label | [LABEL]CCGTGAGGAAAGGTAAGAGATGA{G} |
| | SEQ IDNO 41 | ALAS1_427+12AL_F_Label | [LABEL]ACTCCGTGAGGAAAGGTAAGAGATGA{A} |
| | SEQ IDNO 42 | ALAS1_q7_R | GTTTCTTCGCACCAGAAAGAAAGTCCCA |
| 15 | SEQ IDNO 43 | MRPS9_135+31CL_F_Label | [LABEL]GGAAGACTGGAAGCGGCTTA{C} |
| | SEQ IDNO 44 | MRPS9_135+31TL_F_Label | [LABEL]CATGGAAGACTGGAAGCGGCTTA{T} |

(suite)

| | | | |
|---|---|---|---|
| | SEQ IDNO 45 | MRPS9_q7_R | GTTTCTTAGGTCGCTCCACTTCTACCTTCA |

où les bases entre accolades sont des bases à modification LNA ; [LABEL] est la modification en 5' de l'amorce.

**12.** Procédé selon la revendication 9 ou 11, dans lequel lesdits SNP sont détectés par détermination de la taille desdits amplicons générés par PCR multiplex à spécificité d'allèle, de préférence par un procédé pour la séparation d'ADN basée sur la taille, tel qu'une électrophorèse capillaire.

**13.** Procédé selon la revendication 12, dans lequel ledit test de profilage de SNP dans ladite étape b) est automatisé par un logiciel reconnaissant lesdits produits de PCR multiplex marqués.

**14.** Procédé selon l'une quelconque des revendications 1 à 13, dans lequel ledit NGS est un NGS par capture de cible ou un NGS d'amplicon.

**15.** Trousse pour la détection d'une combinaison d'au moins 8 SNP telle que définie dans la revendication 4, comprenant des amorces telles que définies dans l'une quelconque des revendications 5 à 11, ladite trousse comprenant de préférence en outre :

- des réactifs de PCR multiplex ; et/ou
- des sondes ou amorces oligonucléotidiques de NGS conçues pour capturer ou amplifier des séquences comprenant lesdits au moins 8 SNP.

**16.** Utilisation de la trousse selon la revendication 15 dans un procédé pour valider des résultats de génotypage par NGS d'un panel de gènes testés en série d'au moins 2 patients, selon l'une quelconque des revendications 1 à 14.

**17.** Procédé pour détecter des polymorphismes dans l'ADN d'un patient, comprenant la réalisation, de préférence en parallèle, des deux étapes suivantes :

a) détection de polymorphismes au moyen d'un test par NGS, et
b) validation des résultats d'un génotypage par NGS utilisant le procédé selon l'une quelconque des revendications 1 à 14.

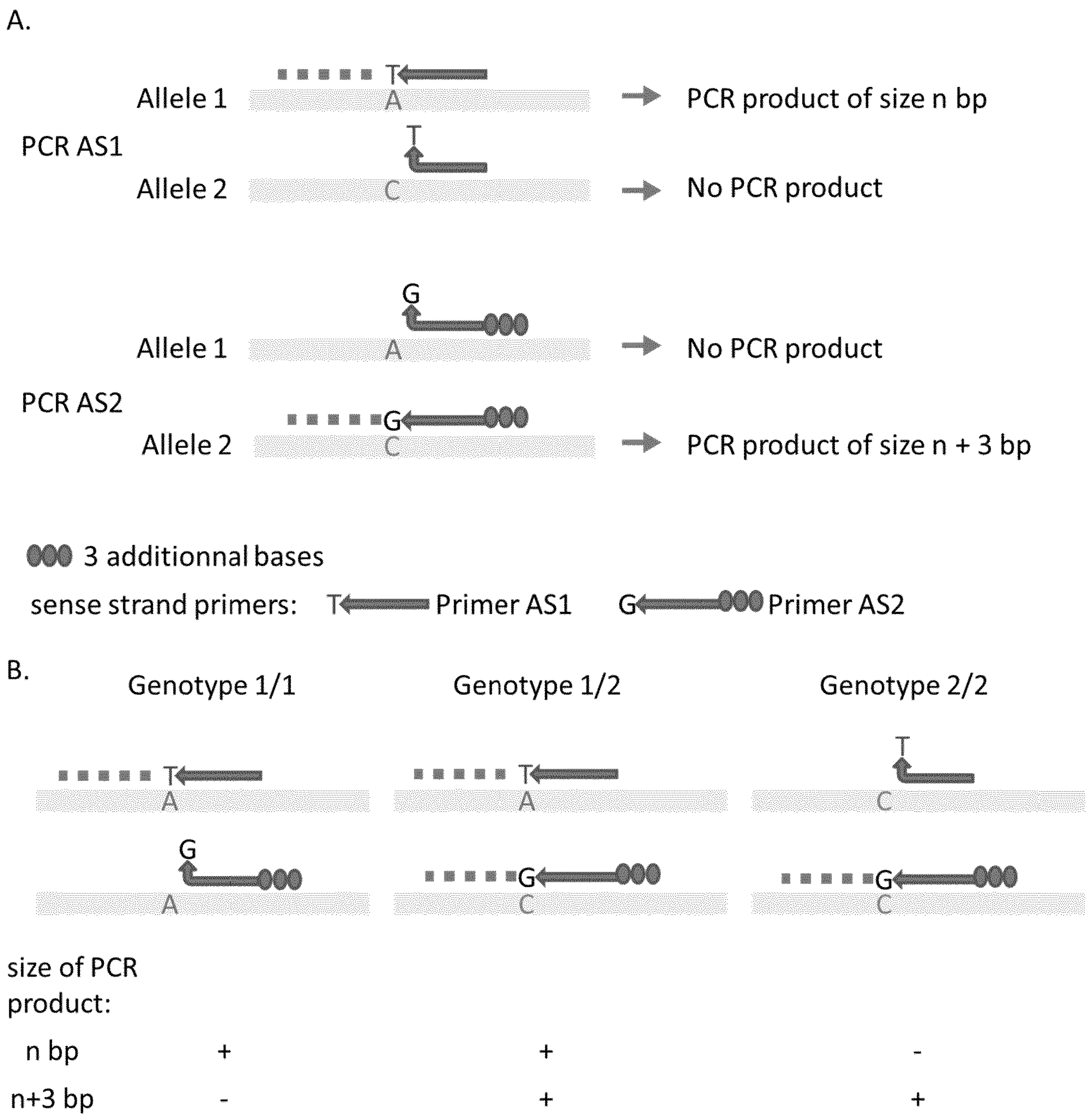
A.
Allele 1
PCR product of size n bp
PCR AS1
Allele 2
No PCR product
Allele 1
No PCR product
PCR AS2
Allele 2
PCR product of size n + 3 bp
T
A
T
C
G
A
G
C
3 additionnal bases
sense strand primers:
Primer AS1
Primer AS2
B.
Genotype 1/1
Genotype 1/2
Genotype 2/2
size of PCR product:
n bp
+
+
-
n+3 bp
-
+
+

Fig. 1

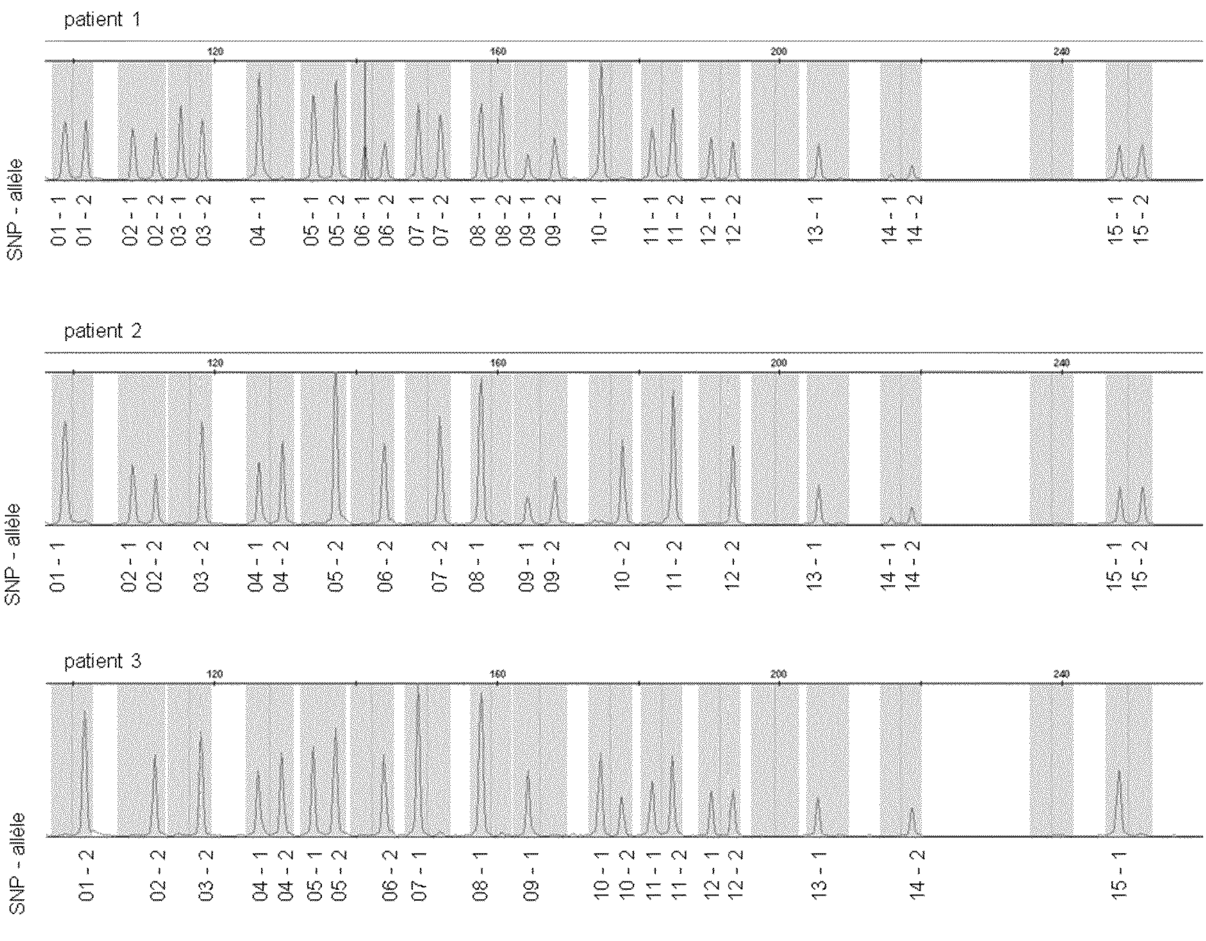


Fig. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 17306106


### Non-patent literature cited in the description


- **AUER et al.** NHLBI GO Exome Sequencing Project. Guidelines for Large-Scale Sequence-Based Complex Trait Association Studies: Lessons Learned from the NHLBI Exome Sequencing Project. *Am J Hum Genet.,* 06 October 2016, vol. 99 (4), 791-801
- **BROWNSTEIN MJ ; CARPENTER JD ; SMITH JR.** Modulation of non-templated nucleotide addition by Taq DNA polymerase: primer modifications that facilitate genotyping. *BioTechniques,* 1996, vol. 20, 1004-1010
- **EISENBERG E ; LEVANON EY.** Human housekeeping genes are compact. *Trends Genet,* 2003, vol. 19 (7), 362-5
- **LEK M et al.** Exome Aggregation Consortium. Analysis of protein-coding genetic variation in 60,706 humans. *Nature,* 18 August 2016, vol. 536 (7616), 285-91
- **MANIATIS N et al.** The first linkage disequilibrium (LD) maps: delineation of hot and cold blocks by diplotype analysis. *Proc Natl Acad Sci U S A.,* 19 February 2002, vol. 99 (4), 2228-33
- **MATTHIJS G ; SOUCHE E ; ALDERS M et al.** Guidelines for diagnostic next-generation sequencing. *Eur J Hum Genet,* 2016, vol. 24, 2-5
- **PENGELLY RJ et al.** A SNP profiling panel for sample tracking in whole-exome sequencing studies. *Genome Med.,* 2013, vol. 5, 89
- **REICH DE et al.** Linkage disequilibrium in the human genome. *Nature,* 10 May 2001, vol. 411 (6834), 199-204
- **RISCH N ; MERIKANGAS K.** The future of genetic studies of complex human diseases. *Science,* 13 September 1996, vol. 273 (5281), 1516-7
- **VOELKERDING KV ; DAMES S ; DURTSCHI JD.** Next generation sequencing for clinical diagnostics-principles and application to targeted resequencing for hypertrophic cardiomyopathy: a paper from the 2009 William Beaumont Hospital Symposium on Molecular Pathology. *J Mol Diagn.,* 2010, vol. 12 (5), 539-551
- **ZHU J1 ; HE F ; SONG S et al.** How many human genes can be defined as housekeeping with current expression data?. *BMC Genomics,* 2008, vol. 9, 172

SEQUENCE LISTING

<110> ASSISTANCE PUBLIQUE - HOPITAUX DE PARIS

<120> METHOD TO CONFIRM VARIANTS IN NGS PANEL TESTING BY SNP GENOTYPING

<130> B374973PCT D37106

<150> EP17306106.0
<151> 2017-08-29

<160> 60

<170> PatentIn version 3.5

<210> 1
<211> 22
<212> DNA
<213> Artificial

<220>
<223> Primer

<220>
<221> LNA-Cytosine
<222> (22)..(22)

<400> 1
cacagccatc cagtgcaaga ac 22

<210> 2
<211> 25
<212> DNA
<213> Artificial

<220>
<223> Primer

<220>
<221> LNA-Thyrosine
<222> (25)..(25)

<400> 2
caacacagcc atccagtgca agaat 25

<210> 3
<211> 29
<212> DNA
<213> Artificial

<220>
<223> Primer

<400> 3
gtttcttaag atgcgctgca ctttagcaa 29

<210> 4
<211> 23
<212> DNA
<213> Artificial

<220>
<223> Primer

<220>
<221> LNA-Adenine
<222> (23)..(23)

<400> 4
agaaacagca attggcctaa gca 23

<210> 5
<211> 26
<212> DNA
<213> Artificial

<220>
<223> Primer

<220>
<221> LNA-Guanine
<222> (26)..(26)

<400> 5
atgagaaaca gcaattggcc taagcg 26

<210> 6
<211> 30

<212> DNA
<213> Artificial

<220>
<223> Primer

<400> 6
gtttcttatt ctcttcctct tccagccaca 30

<210> 7
<211> 20
<212> DNA
<213> Artificial

<220>
<223> Primer

<220>
<221> LNA-Guanine
<222> (20)..(20)

<400> 7
gatctttgca ggccacctcg 20

<210> 8
<211> 23
<212> DNA
<213> Artificial

<220>
<223> Primer

<220>
<221> LNA-Adenine
<222> (23)..(23)

<400> 8
gatgatcttt gcaggccacc tca 23

<210> 9
<211> 29
<212> DNA
<213> Artificial

<220>
<223> Primer

<400> 9
gtttctttga cctgtacccc tgggtttct 29

<210> 10
<211> 21
<212> DNA
<213> Artificial

<220>
<223> Primer

<220>
<221> LNA-Cytosine
<222> (21)..(21)

<400> 10
ggctccagct tgtgtttctg c 21

<210> 11
<211> 24
<212> DNA
<213> Artificial

<220>
<223> Primer

<220>
<221> LNA-Thyrosine
<222> (24)..(24)

<400> 11
ttgggctcca gcttgtgttt ctgt 24

<210> 12
<211> 29
<212> DNA
<213> Artificial

<220>
<223> Primer

<400> 12
gtttcttcca gcctcaggga caagagatt 29

<210> 13
<211> 20
<212> DNA
<213> Artificial

<220>
<223> Primer

<220>
<221> LNA-Cytosine
<222> (20)..(20)

<400> 13
gaggcgctca cgactgtgac 20

<210> 14
<211> 23
<212> DNA
<213> Artificial

<220>
<223> Primer

<220>
<221> LNA-Thyrosine

<222> (23)..(23)

<400> 14
caagaggcgc tcacgactgt gat 23

<210> 15
<211> 26
<212> DNA
<213> Artificial

<220>
<223> Primer

<400> 15
gtttcttacc cctctggagc aggcaa 26

<210> 16
<211> 24
<212> DNA
<213> Artificial

<220>
<223> Primer

<220>
<221> LNA-Guanine
<222> (23)..(24)

<400> 16
gatgtcagtg agcaagtcca gagg 24

<210> 17
<211> 27
<212> DNA
<213> Artificial

<220>
<223> Primer

<220>
<221> LNA-Adenine
<222> (27)..(27)

<400> 17
agagatgtca gtgagcaagt ccagaga 27

<210> 18
<211> 29
<212> DNA
<213> Artificial

<220>
<223> Primer

<400> 18
gtttcttcag tggtcaagtc agggatcgg 29

<210> 19

<211> 21
<212> DNA
<213> Artificial

<220>
<223> Primer

<220>
<221> LNA-Adenine
<222> (21)..(21)

<400> 19
ttgaagctgc acacaggggt a 21

<210> 20
<211> 24
<212> DNA
<213> Artificial

<220>
<223> Primer

<220>
<221> LNA-Guanine
<222> (24)..(24)

<400> 20
tcattgaagc tgcacacagg ggtg 24

<210> 21
<211> 29
<212> DNA
<213> Artificial

<220>
<223> Primer

<400> 21
gtttcttgtc tgcattcctg gaaccagag 29

<210> 22
<211> 23
<212> DNA
<213> Artificial

<220>
<223> Primer

<220>
<221> LNA-Guanine
<222> (23)..(23)

<400> 22
gcatgttctt tgtcatgtgc tcg 23

<210> 23
<211> 26
<212> DNA

<213> Artificial

<220>
<223> Primer

<220>
<221> LNA-Adenine
<222> (26)..(26)

<400> 23
aacgcatgtt ctttgtcatg tgctca 26

<210> 24
<211> 34
<212> DNA
<213> Artificial

<220>
<223> Primer

<400> 24
gtttcttttt acagacatgc acttcctgaa caac 34

<210> 25
<211> 20
<212> DNA
<213> Artificial

<220>
<223> Primer

<220>
<221> LNA-Cytosine
<222> (20) .. (20)

<400> 25
gcccagccct cagtatctgc 20

<210> 26
<211> 23
<212> DNA
<213> Artificial

<220>
<223> Primer

<220>
<221> LNA-Thyrosine
<222> (23) .. (23)

<400> 26
ttcgcccagc cctcagtatc tgt 23

<210> 27
<211> 33
<212> DNA
<213> Artificial

<220>
<223> Primer

<400> 27
gtttcttcca ttgagctgga caagaagtac atc 33

<210> 28
<211> 26
<212> DNA
<213> Artificial

<220>
<223> Primer

<220>
<221> LNA-Guanine
<222> (26) .. (26)

<400> 28
tctggggtaa agagcagtga cttatg 26

<210> 29
<211> 29
<212> DNA
<213> Artificial

<220>
<223> Primer

<220>
<221> LNA-Adenine
<222> (29) .. (29)

<400> 29
acatctgggg taaagagcag tgacttata 29

<210> 30
<211> 28
<212> DNA
<213> Artificial

<220>
<223> Primer

<400> 30
gtttcttcga tgggttgctg gccttcta 28

<210> 31
<211> 30
<212> DNA
<213> Artificial

<220>
<223> Primer

<220>
<221> LNA-Cytosine
<222> (30)..(30)

<400> 31
caagcagaaa gggagaaatt agtaggactc 30

<210> 32
<211> 33
<212> DNA
<213> Artificial

<220>
<223> Primer

<220>
<221> LNA-Thyrosine
<222> (33)..(33)

<400> 32
tgacaagcag aaagggagaa attagtagga ctt 33

<210> 33
<211> 31
<212> DNA
<213> Artificial

<220>
<223> Primer

<400> 33
gtttcttaac cattgcagaa cagctctcca t 31

<210> 34
<211> 19
<212> DNA
<213> Artificial

<220>
<223> Primer

<220>
<221> LNA-Thyrosine
<222> (19) .. (19)

<400> 34
cgcactcgga gccagcagt 19

<210> 35
<211> 22
<212> DNA
<213> Artificial

<220>
<223> Primer

<220>
<221> LNA-Guanine
<222> (21) .. (21)

<220>
<221> LNA-Cytosine

<222> (22) .. (22)

<400> 35
tgacgcactc ggagccagca gc 22

<210> 36
<211> 27
<212> DNA
<213> Artificial

<220>
<223> Primer

<400> 36
gtttctttga tggccatggg aatggat 27

<210> 37
<211> 23
<212> DNA
<213> Artificial

<220>
<223> Primer

<220>
<221> LNA-Thyrosine
<222> (23) .. (23)

<400> 37
ttatcacttg atccagccgc aat 23

<210> 38
<211> 26
<212> DNA
<213> Artificial

<220>
<223> Primer

<220>
<221> LNA-Adenine
<222> (25) .. (25)

<220>
<221> LNA-Cytosine
<222> (26) .. (26)

<400> 38
cagttatcac ttgatccagc cgcaac 26

<210> 39
<211> 32
<212> DNA
<213> Artificial

<220>
<223> Primer

<400> 39
gtttcttgat gggttacacc aggcattact ga 32

<210> 40
<211> 24
<212> DNA
<213> Artificial

<220>
<223> Primer

<220>
<221> LNA-Guanine
<222> (24) .. (24)

<400> 40
ccgtgaggaa aggtaagaga tgag 24

<210> 41
<211> 27
<212> DNA
<213> Artificial

<220>
<223> Primer

<220>
<221> LNA-Adenine
<222> (27) .. (27)

<400> 41
actccgtgag gaaaggtaag agatgaa 27

<210> 42
<211> 28
<212> DNA
<213> Artificial

<220>
<223> Primer

<400> 42
gtttcttcgc accagaaaga aagtccca 28

<210> 43
<211> 21
<212> DNA
<213> Artificial

<220>
<223> Primer

<220>
<221> LNA-Cytosine
<222> (21) .. (21)

<400> 43
ggaagactgg aagcggctta c 21

<210> 44
<211> 24
<212> DNA
<213> Artificial

<220>
<223> Primer

<220>
<221> LNA-Thyrosine
<222> (24) .. (24)

<400> **44**
catggaagac tggaagcggc ttat 24

<210> 45
<211> 30
<212> DNA
<213> Artificial

<220>
<223> Primer

<400> 45
gtttcttagg tcgctccact tctaccttca 30

<210> 46
<211> 201
<212> DNA
<213> Human

<400> 46

**ctgcaagctt tttgctgcgc ctggtaaaga tgcgctgcac tttagcaatt ttgccaaaat 60**

**ggttctccag aatggtcctg tcgttgaggt agtcagggat gttcttgcac tggatggctg 120**

**tgacttcaga gggagacaag cccccaagac tgtctgtgct ctcgcttctg ttactctgac 180**

**gcgccggagt tcctcttaga g 201**

<210> 47
<211> 201
<212> DNA
<213> Human

<400> 47

**ctgtgcaaac ccttcctgta ttctcttcct cttccagcca caaggtagcc aatggccttt 60**

**ccaacggtat agggtgcagg acatcctcct agactgcccc tgcttaggcc aattgctgtt 120**

**tctcctccct ccagggcgga gggctctgaa gctgcagagc tggcagaaat ctatgccaaa 180**

**ctggaggaga ttgaggctga c 201**

<210> 48
<211> 201

<212> DNA
<213> Human

<400> 48

ggaaggggtg acctgtaccc ctgggtttct cacttacact tttctgcttg ttttccagag 60

gggtgatgga gaagtccttt ctggagtatt acgacttcta cgaggtggcc tgcaaagatc 120

gcctgcacct tcaaggccaa actatgcagg taatacaacc cctgctgcta attgcagaag 180

ccctacagct ggccatgtaa a 201

<210> 49
<211> 201
<212> DNA
<213> Human

<400> 49

ttacagcccc cagcgtgggt ggacctgtgt gggtccgtct tggggttccc tcgttgaaca 60

tgctgtcaaa ccaggacact ggctccagct tgtgtttctg ctcttggcca tcgtctggga 120

gtggacatgc aggctatggg ggtggggggc acttagaagg agaaaggcct aaaactggaa 180

tctcttgtcc ctgaggctgg c 201

<210> 50
<211> 201
<212> DNA
<213> Human

<400> 50

tctgtgtgac tgcgtgtctg gacgtctggc tggccgattg gtggacttgc tggctccagt 60

gtttattctt tcccgtattt agaggcgctc acgactgtga catcatagtg attgaatcat 120

tcattttttg tccctttcag gcagttctgc accggagaat tcaggtcagt ttcagatgtg 180

tttgacttct ttgcctgctc c 201

<210> 51
<211> 201
<212> DNA
<213> Human

<400> 51

cttgtatttt ttaggctacc gttgaacaac taatgtttga agagaagaat aaagctcaga 60

gattacagac agaattagat gtcagtgagc aagtccagag ggattttgta aagctttcac 120

agacccttca ggtgaggcat ttggggaacc acatacagag cacgaaggca gttttggggg 180

acagaacctt gccgatccct g 201

<210> 52
<211> 201
<212> DNA
<213> Human

<400> 52

**ccactgtgtc ggagccgggt ctctgggtgc tgacgggcag ctccgcggcc aggctctccc 60**

**cgatgtactt gttctgggag ttgaagctgc acacaggggt atgacgaggg acgggggca 120**

**atggccctcc gttcacaatt tccccgactg acaccgtcag cttcctagta ataaacaccg 180**

**acttcctggt cttggataat c 201**

<210> 53
<211> 201
<212> DNA
<213> Human

<400> 53

**ggggcacgac tgcgtgtgag cactatattc tgaatccaca actgctgcag tcgggcctga 60**

**ggaggaacgg agcagaatgc ctcttacctg cagcaaaggc cgagcacatg acaaagaaca 120**

**tgcccacggc gatcctcttc agggaggatg ggagcaggcc atgtcttctc aaaatgggat 180**

**cgaccagttt gtccttcaga g 201**

<210> 54
<211> 201
<212> DNA
<213> Human

<400> 54

**ccgggctgcc agcaacatgg cactgggcaa gttccgggcc gcgctgcgag actacgagac 60**

**ggtgagctgg ggagtgggcc aggcctggca cctgagccag gcagatactg agggctgggc 120**

**tggcggggac gggtgtgagg agccattcac tcctctactt accactcagc cagccagcaa 180**

**gtgcctgtgg ggccttgggc c 201**

<210> 55
<211> 201
<212> DNA
<213> Human

<400> 55

gtttattcca cgcctcggta gggaccaata catagtccaa ttcatcaatt aagtgttctt 60

tcaaggtctg actctcagga tctaaaaaag gaaaagagca cataagtcac tgctctttac 120

cccagaaatg atggttttac atgatttcca tgtacttttc agaaataaga aaattattta 180

cagaaagaaa caggaatgaa a 201

<210> 56
<211> 201
<212> DNA
<213> Human

<400> 56

cctccaaccc tttccttccc ttttaacccc ccttcttctc cctcccctgg atctcaagtt 60

ttccacctat ctctttcttg cgtttagcac tctccatagt gagtcctact aatttctccc 120

tttctgcttg tctcccttgt ctctccttag taagcgatac gaatcccagc ttcgggatct 180

tgagcggcag tcagagcaac a 201

<210> 57
<211> 201
<212> DNA
<213> Human

<400> 57

ggatgagtgt gcccgaagcc ccccaccctg cacctacggc cggtgtgaga acacagaagg 60

cagcttccag tgtgtctgcc ccatgggctt ccaacccaac actgctggct ccgagtgcga 120

gggtgaggcc ggggagggag ggaggagtgt ggatgggtga ggggggagtt ggaccacttc 180

ttcaaggcca ccctctcccc t 201

<210> 58
<211> 201
<212> DNA
<213> Human

<400> 58

aaggggtact ccttatgcta gcagattcaa agatatgcct aactttattg cccttgagaa 60

gtcatcagtt ctccgccact gctgtgacct tttgataggc attgcggctg gatcaagtga 120

taagatttgc accagcagtc tccaagttca gagacgattc aaggcaatga tggcatctat 180

tggaagactt tcacatggtg a 201

<210> 59
<211> 201
<212> DNA
<213> Human

<400> 59

```
acagatgaat cagagaggca gcagtgtctt ctgcaaagcc agtcttgagc ttcaggagga    60
tgtgcaggaa atgaatgccg tgaggaaagg taagagatga gttgtgaacc attagtggta   120
gtgaaggggt ccttttagtt cttttgggcc ctcagatttg tgtatgagct attattaggg   180
cagtatttat ggaaacacct t                                             201
```

<210> 60
<211> 201
<212> DNA
<213> Human

<400> 60

```
tagcctcgcc cggaagcaag gcctctggaa aaccgcggcc cctgagttgc aaacaaatgt    60
cagatcccag gtaaggcctg ggaagactgg aagcggctta cctctgccgc gcgaggatgt   120
ggagccagcg cggacacctt cccccagcgt ctcgcgtgcc ttcaggcagg gactctagga   180
tcttaggtat ttagtggagg t                                             201
```